# EUROPEAN PATENT SPECIFICATION

(11) **EP 4 259 108 B1**
(45) Date of publication and mention of the grant of the patent: **11.06.2025**
(21) Application number: 21839100.1
(22) Date of filing: 13.12.2021
(51) Int. Cl.: A61K 9/50, A61K 31/573, A61K 38/43

(54) **SUSTAINED RELEASE FORMULATIONS OF CRYSTALLINE DRUGS**
FORMULIERUNGEN KRISTALLINER ARZNEIMITTEL MIT VERZÖGERTER FREISETZUNG
FORMULATIONS DE MÉDICAMENTS CRISTALLINS À LIBÉRATION PROLONGÉE

(30) Priority: 11.12.2020 GB 202019594
(43) Date of publication of application: 18.10.2023
(73) Proprietor: Queen Mary University of London, London, Greater London E1 4NS (GB)
(72) Inventor: GOULD, David James, London Greater London E1 4NS (GB); READ, Jordan Elise, London Greater London E1 4NS (GB); SUKHORUKOV, Gleb, London Greater London E1 4NS (GB); KUDRIAVTCEVA, Valeriia, London Greater London E1 4NS (GB); ZHANG, Jiaxin, London Greater London E1 4NS (GB)
(74) Representative: Leonard, Thomas Charles
(86) International application number: PCT/EP2021/085569
(87) International publication number: WO 2022/123089

(56) References cited:
- WO-A1-2019/214726
- WO-A2-2009/042231
- US-A1- 2004 115 279
- MCHUGH K.J. ET AL.: "Fabrication of fillable microparticles and other complex 3D microstructures", SCIENCE, vol. 357, no. 6356, 15 September 2017 (2017-09-15), US, pages 1138 - 1142, XP055896326, ISSN: 0036-8075, Retrieved from the Internet <URL:https://www.ncbi.nlm.nih.gov/pmc/articles/PMC6510330/pdf/Science-357-1138.pdf> DOI: 10.1126/science.aaf7447

## Description

The present application relates to sustained release formulations of crystalline drugs, including corticosteroids, in particular dexamethasone and prednisolone.

### BACKGROUND

In treatment of chronic diseases, therapeutics are usually delivered long-term. Where the disease is localised at a specific site, it can be advantageous to deliver the therapeutic directly, to have a therapeutic effect with a lower drug dose and to avoid off-target effects of systemically delivered drugs. In the case of disorders affecting joints, these benefits, along with the aging population and growth in patient numbers, have led to a forecasted 8% compound annual growth rate for the global joint pain injections market that is predicted to reach US$ 5.7 billion by 2026. However, this uptrend and the associated burden on health services now means there is an urgent clinical need to develop treatment options that increase the active length of time of these drugs.

A broad example are glucocorticoids, an established treatment for joint inflammation and a class of steroid hormones that have been used in the clinic in excess of 70 years. Glucocorticoids are potent drugs, but when they are delivered systemically for a prolonged period of time they cause multiple side effects, including weight gain, osteoporosis, moon face, diabetes and also immunocompromise patients. To avoid these systemic side effects, many synthetic glucocorticoids have been formulated for local delivery, with two main types of formulation. One type of formulation uses a mixture of polymer and drug that is intended to slowly release as the drug diffuses out and the polymer degrades. Alternatively, the other type of formulation makes use of crystalline suspensions of the drug that are intended to slowly dissolve to achieve a prolonged therapeutic effect.

A recent example is Zilretta^{®} (triamcinolone acetonide extended-release injectable suspension) produced by Flexion Therapeutics, which is a mixture of the steroid triamcinolone acetonide and polymer PLGA. Zilretta^{®} was approved for treatment of pain in knee osteoarthritis by the FDA in 2017. Whilst Zilretta^{®} is intended to slowly release as the drug diffuses out and the polymer degrades, clinical trial data (ClinicalTrials.gov Identifier: NCT02637323, Study of FX006 for the Treatment of Pain in Patients With Osteoarthritis of the Knee), shows that after an injection of 32 mg there is a burst (rapid) release of the drug that ultimately curtails the active length of the time of the intervention, with concentration of drug in the synovial fluid reducing to only 3590 pg/ml after 6 weeks (from a high of around 235,000 pg/ml) and 98% of the drug being released in that time. If the steroid release profile could be optimised to release at levels of 10,000 pg/ml in the synovial fluid - avoiding a burst release, but maintaining a level well above that at 6 weeks where a therapeutic effect is still seen - then a dosage of 32 mg could last for up to 72 weeks. Further, the problem of burst-release is not just seen with the polymer-drug formulation. During the Zilretta^{®} clinical trial, the polymer-drug formulation was compared to an approved steroid crystal suspension of the same drug - Kenalog^{®}-40 (triamcinolone acetonide). The starting dose was greater at 40 mg, and because less drug (7.7 pg/ml) was measured in the synovial fluid after 6 weeks, the burst (rapid) release must be even greater.

As such, both polymer and crystal-suspension formulations suffered from a curtailed drug-release profile due to the burst (rapid) release seen immediately after delivery. Importantly, this is just one example in a market where there are already a number of FDA-approved polymer formulations, even when just considering the polymer PLGA (Park, K. et al. Injectable, long-acting PLGA formulations: Analyzing PLGA and understanding microparticle formation. Journal of Controlled Release 304, 125-134 (2019)), that have applications in conditions ranging from schizophrenia (Perseris^{™}), through to the treatment of drug addiction (Vivitrol^{®} (naltrexone) and Sublocade^{™} (buprenorphine extended-releases)), and where crystal suspensions, such as methylprednisolone acetate (Depo-Medrol^{®}), are used to treat a wide variety of disorders, including rheumatic disorders, allergies, asthma, croup, COPD and multiple sclerosis.

To further reinforce the inadequacy of current technology for long term drug delivery, polymer-mixing and crystal suspensions are generally more suited to hydrophobic molecules, so less applicable to hydrophilic drugs (such as protein therapeutics).

Thus, there is huge scope to improve on the active length of time of long-term delivered drugs, and in doing so, improve the quality of life for patients with chronic diseases and the burden on health services.

The present invention provides new sustained-release formulations, in particular for corticosteroids such as dexamethasone.

### SUMMARY OF THE INVENTION

The invention relates to microencapsulated crystalline drug compositions.

In a first aspect of the invention, there is provided a sustained-release composition comprising a plurality of microcapsules, wherein the microcapsules comprise a core and a shell, wherein the core comprises a crystalline drug and the shell comprises polylactic acid (PLA).

In a second aspect of the invention, there is provided a sustained-release composition comprising a plurality of microchambers, wherein the microchambers comprise a core and a shell, wherein the core comprises a crystalline drug and the shell comprises polylactic acid (PLA).

In a third aspect of the invention there is provided a method for micro-encapsulating a drug, comprising:
providing a first stamp comprising a plurality of microwells, wherein the microwells of the first stamp are coated with a polymer composition comprising polylactic acid (PLA);
loading crystalline drug into the coated microwells;
providing a second stamp, wherein the second stamp is planar and is coated on at least one side with the polymer composition comprising polylactic acid (PLA); and
pressing the coated sides of the first and second stamps together to encapsulate the crystalline drug in the wells with the polymer composition to form a film of microchambers.

In a fourth aspect of the invention there is provided a method for micro-encapsulating a drug, comprising:
providing a first stamp comprising a plurality of microwells, wherein the microwells of the first stamp are coated with a polymer composition comprising polylactic acid (PLA);
loading crystalline drug into the coated microwells;
providing a second stamp, wherein the second stamp is planar and is coated on at least one side with the polymer composition comprising polylactic acid (PLA);
pressing the coated sides of the first and second stamps together at a pressure of up to about 0.25 MPa to encapsulate the drug crystals in the wells in the polymer composition to form a film of microchambers;
separating the first and second stamps;
separating the microchambers into separate microcapsules in the wells of the first stamp by removing excess polymer composition from the first stamp; and
removing the microcapsules from the first stamp.

The step of removing the microcapsules drug from the first stamp may comprise:
providing a planar substrate coated with a soluble adhesive;
pressing the planar substrate onto the first stamp to adhere the microcapsules to the slide;
removing the microcapsules from the microwells of the first stamp by separating the planar substrate and the first stamp; and
dissolving the soluble adhesive to release the microcapsules.

In a fifth aspect of the invention there is provided a method for micro-encapsulating a drug, comprising:
providing a first stamp comprising a plurality of microwells, wherein the microwells of the first stamp are coated with a polymer composition comprising polylactic acid (PLA);
loading crystalline drug into the coated microwells;
providing a second stamp, wherein the second stamp is planar and is coated on at least one side with the polymer composition comprising polylactic acid (PLA); and
pressing the coated sides of the first and second stamps together at a pressure of up to about 0.25 MPa and heating the polymer composition to a temperature equal to or greater than the melting point of the polymer composition to encapsulate the drug crystals in the wells in the polymer composition to form a plurality of microparticles;
separating the first and second stamps; and
removing the microcapsules from the first stamp.

The step of removing the microcapsules drug from the first stamp may comprise:
providing a planar substrate coated with a soluble adhesive;
pressing the planar substrate onto the first stamp to adhere the microcapsules to the slide;
removing the microcapsules from the microwells of the first stamp by separating the planar substrate and the first stamp; and
dissolving the soluble adhesive to release the microcapsules.

In a sixth aspect of the invention there is provided a sustained-release composition comprising a film of microchambers obtained or obtainable according to the methods of the third aspect of the invention.

In a seventh aspect of the invention there is provided a sustained-release composition comprising a plurality of microcapsules obtained or obtainable according to the methods of the fourth or fifth aspects of the invention.

In a further aspect of the invention, there is provided a method of treating a disease or disorder, comprising administering a sustained release composition of the invention or administering a film of microchambers of the invention, or administering a plurality of microcapsules of the invention, to a subject in need thereof.

In a further aspect of the invention, there is provided the use of a plurality of microcapsules of the invention in the manufacture of a sustained-release medicament to treat a disease or disorder. In a further aspect of the invention, there is provided a plurality of microcapsules of the invention for use in the treatment of a disease or disorder.

In a further aspect of the invention, there is provided the use of a film of microchambers of the invention in the manufacture of a sustained-release medicament to treat a disease or disorder. In a further aspect of the invention, there is provided a film of microchambers of the invention for use in the treatment of a disease or disorder.

In a still further aspect of the invention there is provided a drug-eluting implantable medical device comprising a film of microchambers, the microchambers each comprising a core and a shell, wherein the core comprises a crystalline drug and the shell comprises polylactic acid (PLA). The present invention also provides a method of preparing the drug-eluting implantable medical device, the method comprising providing an implantable medical device and affixing a film of microchambers to the implantable medical device, the microchambers each comprising a core and a shell, wherein the core comprises a crystalline drug and the shell comprises polylactic acid (PLA).

In a further aspect of the invention, there is provided a method of surgery, comprising implanting a film of microchambers of the invention, or the drug-eluting implantable medical device of the invention, into a patient.

### BRIEF DESCRIPTION OF THE FIGURES

**Figure 1****:** Schematic illustration of the preparation of microchamber array films.
**Figure 2****:** SEM images of some of the micropatterned master stamps utilized to prepare microchambers and microcapsules. A range of different stamps was used.
**Figure 3****:** Process of microchamber assembly with crystal cargo. SEM images of the microchamber arrays film with cylindrical shape microchambers (cylinder 1) made of PCL, where the loaded crystals were model drug 5,6-carboxyfluorescein.
**Figure 4****:** SEM images of the cylindrical microchamber arrays films (cylinder 1) made of PLA encapsulated with dexamethasone crystals.

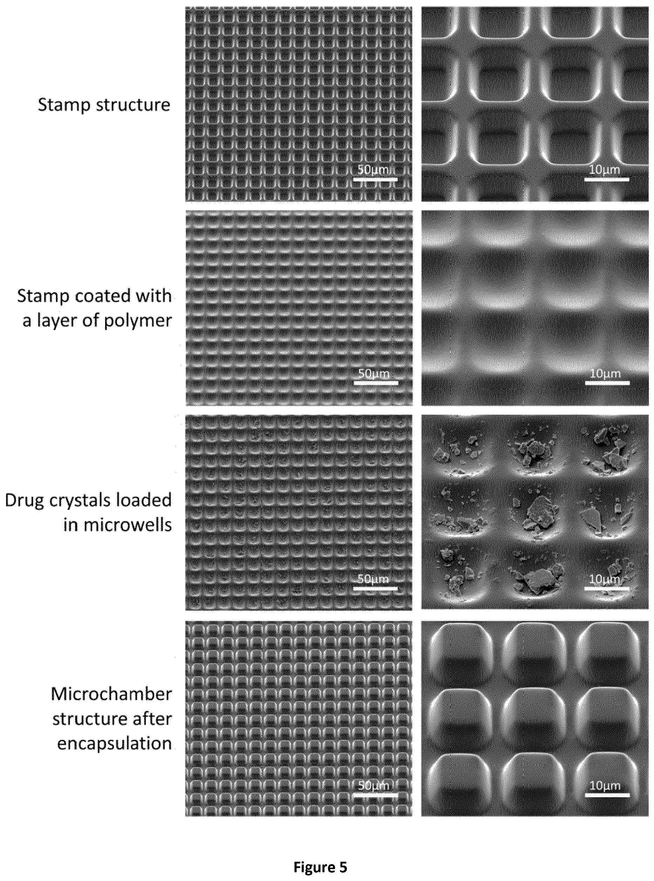

**Figure 5****:** The TP PDMS stamp with the microwell arrays is used for micro-encapsulating crystalline drugs. A layer of polylactic acid (PLA, GoodFellow) film is coated onto the stamp surface by dipping the PDMS stamp into PLA chloroform solution of 1.5% to 3%, while still leaving microwell spaces for loading crystalline drugs. The drug crystals are physically loaded into microwells by spreading drug crystals onto the precoated PDMS surface followed by removing the excess crystals, with only few located near to the ridges between microwells. After that, a glass slide that is also dip-coated with a layer of PLA and dried for 10 seconds is covered and pressed on the top of the PDMS stamp at the pressure of 0.25 - 1 MPa for 1 minute, thus the crystalline drugs are well-sealed and encapsulated into microchamber array films. The film can be easily detached from both first and second stamp to harvest free-standing microchamber array films.
**Figure 6****:** Construction of microchambers with different polymers. SEM images of TP shape microchamber array films made of different biodegradable polymer. By using different polymer solution in the preparation process, TP microchamber array films of PLGA, PLA, PLA-PCL (blends) = 9:1, PLA-PCL = 8:2 and PLA-PCL = 7:3 can be prepared.

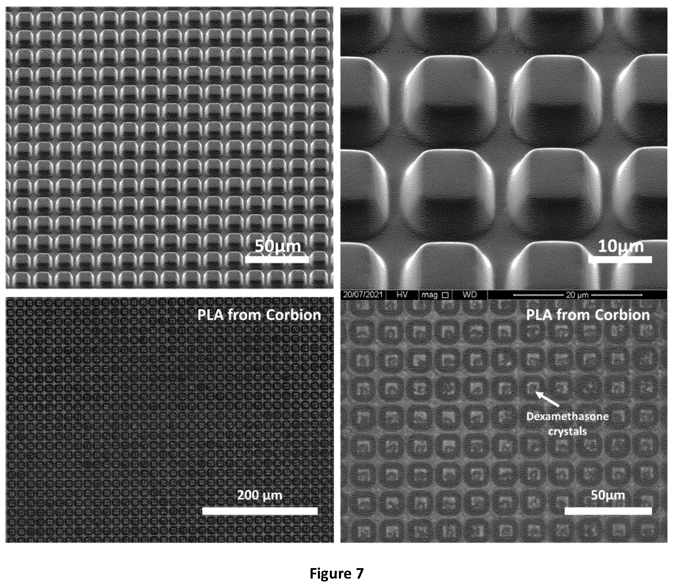

**Figure 7****:** Microchambers prepared with medical grade polymer. The SEM and optical images of TP shape microchamber array film made of medical (GMP) grade PLA. The process for preparing microchamber array films also works when using medical degrade polylactic acid (PURASORB PDL 05, Corbion). As shown in the SEM and optical images above, the surface morphology of TP microchamber arrays film made of medical grade polymer is exactly the same as natural polylactic acid from GoodFellow. Dexamethasone crystals encapsulated into the microchamber are clearly observed in the transmitted optical images.
**Figure 8****:** Drug crystal preparation. SEM images of dexamethasone crystals before and after the physical homogenization.
**Figure 9****:** Optical images of dexamethasone crystals before and after the physical homogenization. The amount of loaded drug crystals can be enhanced by decreasing the size of drug crystals using a homogenizer. Figure 8 and 9 show the size and structure of dexamethasone crystals before and after the homogenization for different times. Each time, the crystals are homogenized at the speed of 5000 for 30 seconds with a Precellys homogenizer.
**Figure 10****:** Preparation of protein crystals. SEM image of catalase from bovine liver powder (Sigma Aldrich, 2,000-5,000 units/mg protein) before treatment and after treatment for 1 minute with Precellys homogenizer.
**Figure 11****:** The morphologic comparison of catalase crystals C30 (Sigma Aldrich, 10,000-40,000 units/mg protein) before homogenization and after homogenization.
**Figure 12****:** SEM image of PDMS stamp of TP shape microwells precoated with a layer of PLA before (top) and after (bottom) the loading of catalase crystals.
**Figure 13****:** SEM image of PDMS stamp of cuboid shape microwells precoated with a layer of PLA before (top) and after (bottom) the loading of crystal catalyses. Figures 12 and 13 show the loading of crystal catalase onto different PDMS stamp (TP above Cuboid below) precoated with a thin layer of PLA film. The catalase crystals were physically spread onto the top of precoated PDMS stamp. After removing the excess crystals, the microwells were occupied by the crystals with the majority of the crystals located inside the microwells.
**Figure 14****:** Microchambers prepared with dexamethasone. The optical images of PLA microchamber array (TP) films loaded with dexamethasone crystals (top) and without drug loading dexamethasone crystals (bottom).

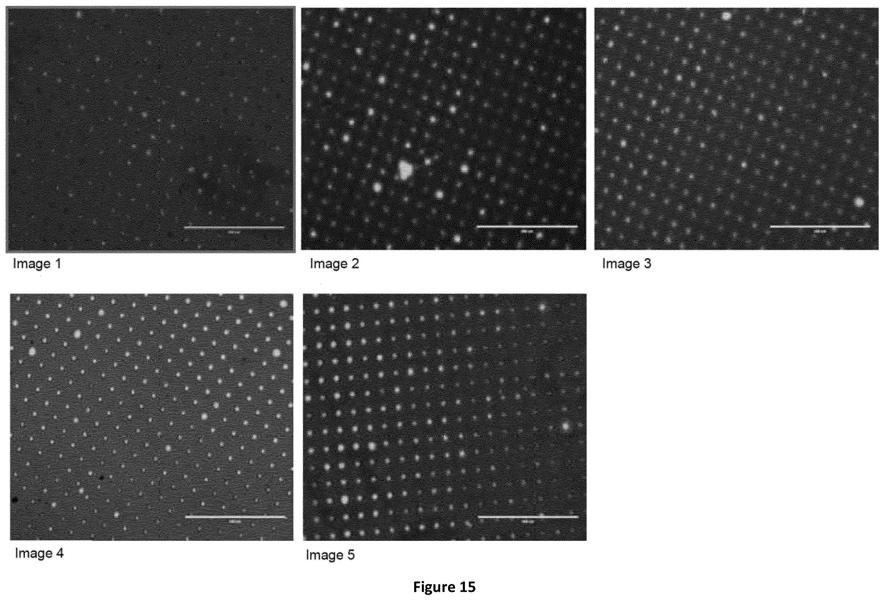

**Figure 15****:** Release of dexamethasone from microchambers. Fluorescence microscopy images of the dexamethasone FITC microchamber film (C1) taken between the start and seventh week of the release experiment.
**Figure 16****:** Weekly release of dexamethasone from the microchamber film array. Data from experiment 2 and inset weekly release values and summary statistics.
**Figure 17****:** Release of dexamethasone from the PLA microchamber film array over the course of 24 weeks.
**Figure 18****:** Schematic illustration of the microchamber array film release experiment setup for data collected in Figures 19 and 20. Media (1 ml) was changed after the first day and then at weekly intervals and was assayed with the responsive cell line.
**Figure 19****:** Weekly release of dexamethasone from the TP shape microchamber film arrays made of PLA (n=4). Similar to data collected with C1 microchambers (Figures 15 to 17) low level release of dexamethasone was detected in the 12 week duration of the experiment.
**Figure 20****:** The cumulative release of dexamethasone from TP shaped microchamber film arrays made of PLA (n=4), PLGA(n=4) and PLA empty. PLGA which degrades more rapidly than PLA resulted in a more rapid release rate.

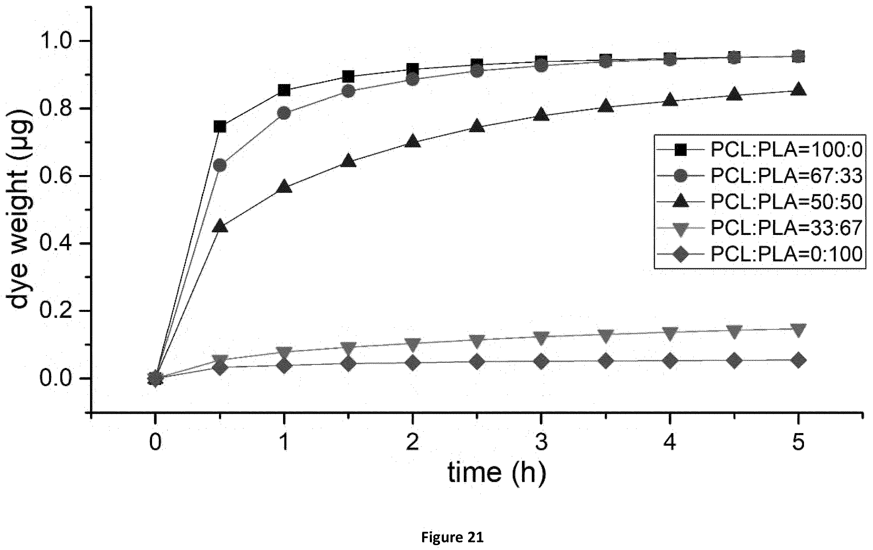

**Figure 21****:** The diffusion curves of PCL blends with PLA in different ratios in PBS loaded with carboxyfluorescein.

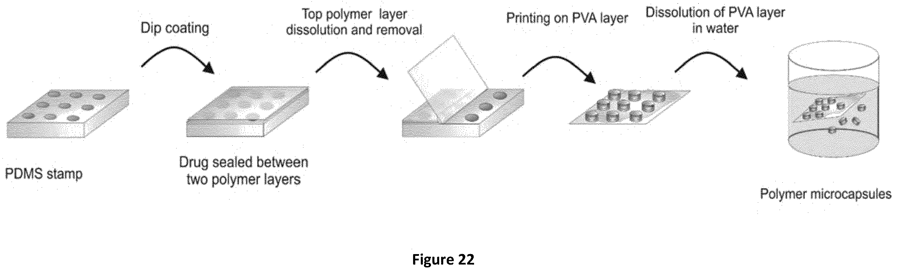

**Figure 22****:** Polymer microcapsule preparation scheme (pressure method).

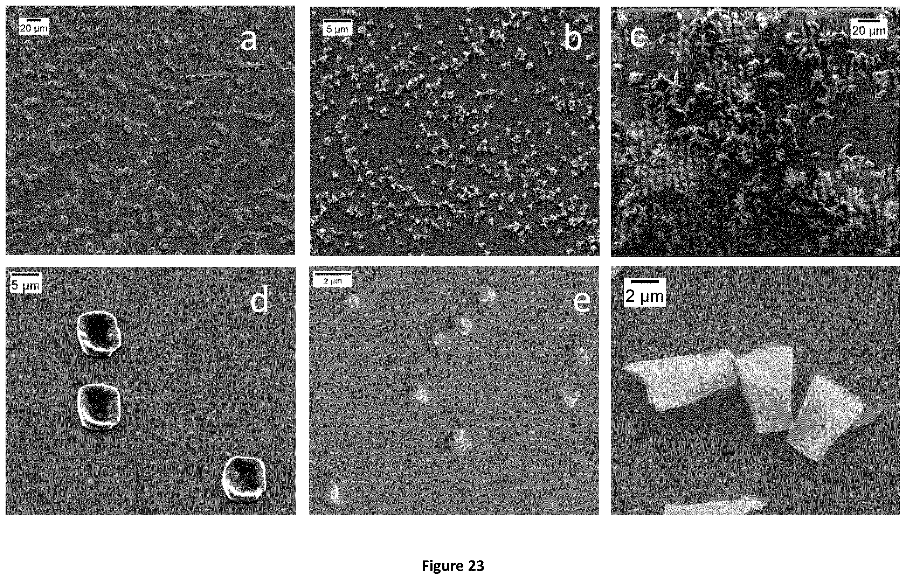

**Figure 23****:** Types of microcapsules prepared with the pressure method. SEM images of PLA (2% in chloroform, Sigma Aldrich) microcapsules A), d) with rectangular shape 14×9×4 µm size, b),e) square based pyramids with side length and height 1.8×2 µm, e, f) blunted cones 9 µm tall, with 5 µm diameter of the larger base and smaller base shaped as a square having 3 µm edge. SEM (ESEM Quanta 400 FEG, FEI, USA) was used to investigate the morphology of obtained samples with imaging conditions of 10 kV accelerating voltage and 10 mm working distance. Prior to the investigation, samples were washed with deionized water and a 5-10 µL drop of each sample was placed on a mounting stage covered with carbon tape and left to dry overnight. And were coated with a thin ~10 nm gold layer (Agar Auto Sputter Coater, Agar Scientific, UK).
**Figure 24****:** SEM images of PLA microcapsules with different shape.
**Figure 25****:** Confocal laser scanning microscopy (CLSM, ZEISS LSM710, Germany) of PLA rectangular microcapsules labeled with Nile Red (red) and loaded with carboxyfluorescein (CF) powder (green). Figure shows a single rectangular capsule, allowing the visualization of PLA shell (a1 in red), the loaded CF (a2 in green), bright field (a3) and the overlap image of the channels (a4).

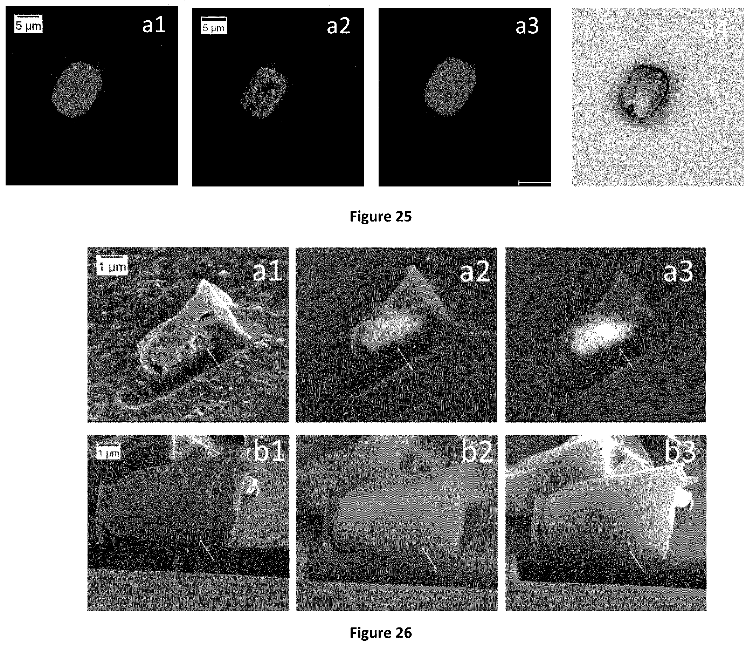

**Figure 26****:** All cross-sections were prepared and imaged with sample stage tilted to 52° to obtain the 90° angle for FIB milling using Ga- ions at 30kV accelerating voltage and 50-300 pA current. SEM imaging was accomplished using Secondary Electron (SE) and Backscattered electron (BSE) detectors. BSE detector was used in order to provide a clear contrast between organic capsule material and a non-organic filler with imaging conditions of 30 kV accelerating voltage and 20-100 µA current. FIB-SEM cross-sectional image of capsules filled with a) FeCl₂ crystals and b) Fe₃O₄ nanopowder obtained with (1) SE, (2) BSE detector and (3) overlaid image. White arrows indicate the cargoes crystals inside the capsules, while red arrows indicate the shell polymer layer.
**Figure 27****:** SEM images of PLGA (10% in acetone, Sigma Alrdrich, lactide:glycolide (75:25), mol wt 66,000-107,000) microcapsules with cuboid shape and size of 11×11×22 µm prepared with cuboid stamp C.
**Figure 28****:** CLSM images PLGA capsules (red) with encapsulated catalase (green) on a) day 0, b) day 1, c) day 3, d) day 6, e) day 14, f) after ultrasonication with a Fisherbrand^{™} Model 120 Sonic Dismembrator with 40% amplitude for 120 seconds on ice.
**Figure 29****:** Release of catalase from pressure microcapsules. Bovine liver catalase (Sigma Aldrich) activity from untreated and sonicated PLGA microcapsules (150 000 capsules per mL) in 1 mL. The catalase activity was measured by Amplex ^{™} Red assay and the number of capsules was determined with hemocytometer. Sonication was done by Fisherbrand^{™} Model 120 Sonic Dismembrator with 40% amplitude for 120 seconds on ice. This data shows that the pressure method can be used to encapsulate enzyme (protein) and upon release it remains active.
**Figure 30****:** Schematic illustration of the preparation of microcapsules. Microcapsules made by the heat method.
**Figure 31****:** SEM images of key stages during the preparation of microcapsules. a, b) polydimethylsiloxane (PDMS) stamp with microwell arrays structure. c, d) a layer of precoated PLA film on the surface of PDMS stamp. e, f) carboxyfluorescein (CF) particles loaded into microwell arrays of PLA films on PDMS stamp. g, h) microcapsules embedded inside microwell arrays after detaching the flat PDMS. i, j) Transferred PLA microcapsules on gelatin. k, l) printed PLA microcapsules after dispersing and wash steps.

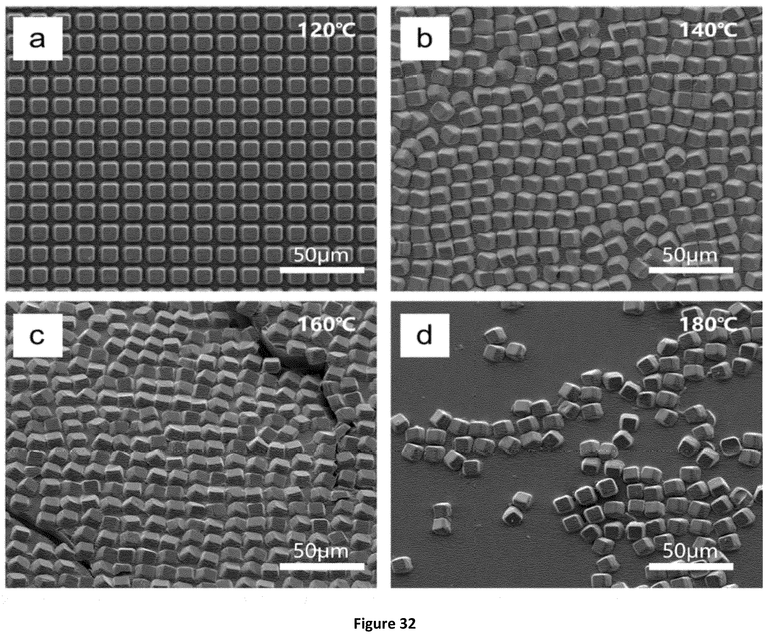

**Figure 32****:** After the sandwiched structure of PDMS template - polymer films - flat PDMS being heated and pressed at different temperatures and lifting over the flat PDMS, the resulted structure transferred onto glass slide by gelatin. a) Heated and pressed at 120°C. The PLA polymer films were not separated by the wall of microwells due to the low temperature. b) Heated and pressed at 140°C. The temperature reached the melting temperature of PLA, thus the PLA films were separated and the individual microcapsules were formed. c) Heated and pressed at 160°C. The results were similar to b) that the individual microcapsules were successfully printed. d) Heated and pressed at 180°C. The temperature was so high that some of the PLA melted and attached onto the flat PDMS. Only some of the microcapsules were formed and transferred onto glass slide by gelatin.
**Figure 33****:** The SEM images of microcapsules made by chloroform solutions of 1.5% PLA, 1.75% PLA and 2% PLA
**Figure 34****:** CLSM images of PLA microcapsules for confirming the encapsulation of CF model drugs. a-d) CF loaded PLA microcapsules printed and embedded inside the microwell arrays of patterned PDMS stamp. e-h) CF loaded printed PLA microcapsules dispersed in DI water. i-l) higher magnification of eh) focusing on one single PLA microcapsule. PLA is labelled with red fluorescent dye Nile Red and the green color represents the encapsulated fluorescent dye particles CF.
**Figure 35****:** SEM images of the morphology of cuboid shape microcapsules. The figures show the specification of the microcapsules of TP and cuboid shape microcapsules, respectively. Both microcapsules have smooth and intact morphology without any dexamethasone crystals exposed.

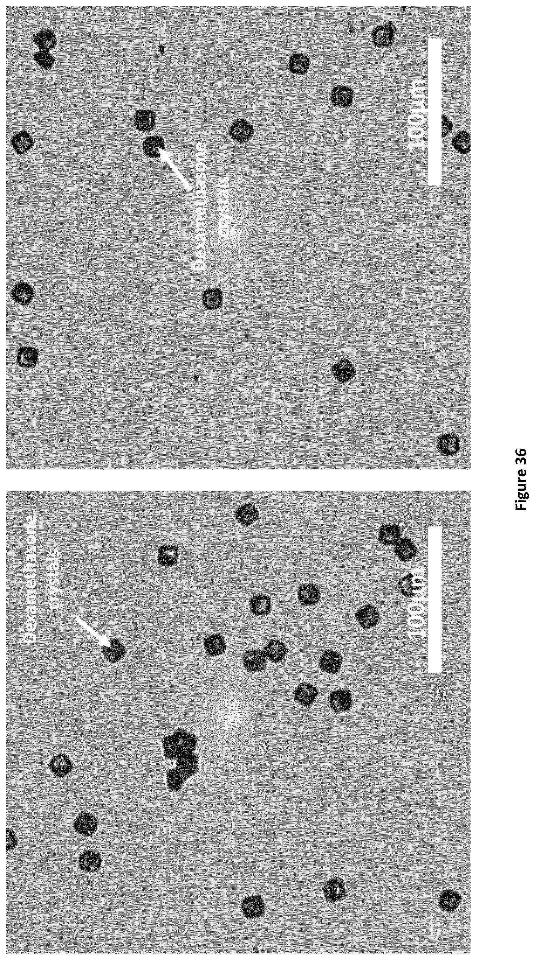

**Figure 36****:** Optical images of TP shape microcapsules loaded with dexamethasone crystals.

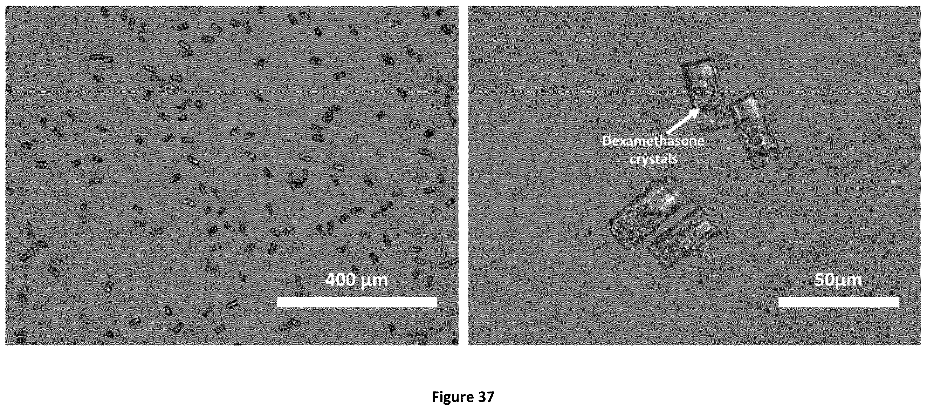

**Figure 37****:** Optical images of cuboid shape microcapsules loaded with dexamethasone crystals.
**Figure 38****:** CLSM images of TP shape microcapsules. The green signal is from the dexamethasone-fluorescein, while the red signal is from PLA polymer which labelled with fluorescent dye Nile red.
**Figure 39****:** CLSM images of TP shape microcapsules. The green signal is from the dexamethasone-fluorescein, while the red signal is from PLA polymer which labelled with fluorescent dye Nile red.

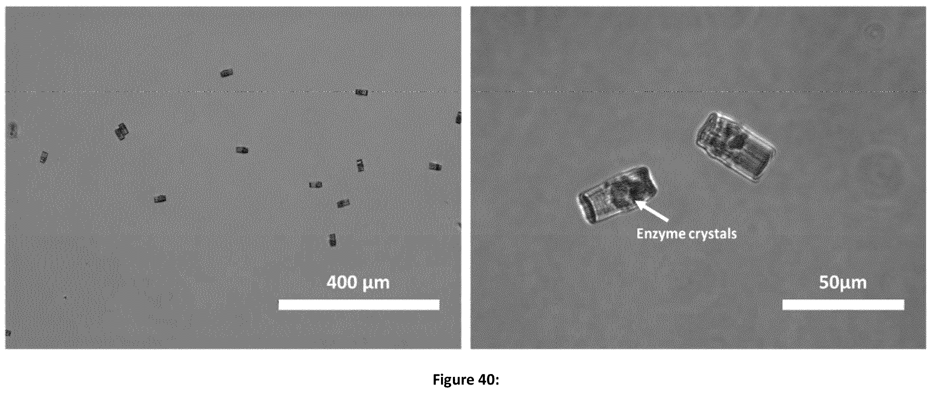

**Figures 40** **&** **41****:** Heat microcapsules with catalase. The optical image and SEM images of PLA microcapsules loaded with catalase crystals are shown in Figure 40 and Figure 41, respectively. Generally, the enzyme loaded PLA microcapsules have exactly same morphology as the dexamethasone crystal loaded ones. Moreover, the optical images also support that the crystal enzymes were well encapsulated inside microcapsules.
**Figure 42****:** Schematic illustration of the microcapsule release experiment setup. Media (1ml) was changed after the first day and then at weekly intervals and was assayed for dex content using the responsive cell assay.
**Figure 43****:** Percentage of dexamethasone released in 3 weeks from dexamethasone crystals (2.6 µg) without encapsulation, 100,000 TP microcapsules (estimated total dexamethasone 7.05 µg) in cell culture media (n=3) or human synovial fluid (n=1). In the group of dexamethasone crystals, almost 95% of the dexamethasone was released within the first 24 hours, and the dexamethasone was essentially exhausted at day 7. In contrast, only 2% of dexamethasone was released after 21 days from TP microcapsules.
**Figure 44****:** Cumulative release of dexamethasone into water at 37°C and 60°C. 10,000 microcapsules in 500 µl of water incubated at 37 or 60°C. 50 µl collected and volume replaced with water. Data shows that low level release observed at 37 °C can be expedited by incubation at 60°C. Dexamethasone quantitated using the cell assay.

### DETAILED DESCRIPTION OF THE INVENTION

The invention provides sustained-release formulations of micro-encapsulated crystalline drugs as well as methods for their preparation and their use in medicine and as components of drug-eluting implantable medical devices. More details of the various aspects of the inventions are now provided.

### Sustained release compositions

The sustained-release compositions may be provided in one of two main formats. The first is in the form of microcapsules and the second is in the form of microchambers. The core-shell structure is the same for both formats, and all optional or preferred features for the microcapsules and microchamber embodiments are equally applicable. However, microcapsules are generally provided in the form of individualized, separate compartments. The microcapsules may therefore be provided in the form of a dispersion of microcapsules (although some microcapsules may aggregate together in a given composition). The dispersion may be in solution or in solid. For example, the dispersion may be in an aqueous solution. Alternatively the dispersion may be in a pharmaceutically acceptable excipient or diluent, such as PBS. The microcapsules can also be provided as a powder of microcapsules, for example as freeze-dried microparticles, with or without pharmaceutically acceptable excipient or diluent.

In contrast, microchambers are provided in the form of a film or single layer of connected microchambers. Connected means the microchambers are attached to other. The microchambers exist in a continuous medium of polymer. The polymer forms the shells of adjacent microchambers. The polymer also forms the connections between adjacent polymers. A composition provided in the form of microcapsules may include some microcapsules that have agglomerated together, but the skilled person would understand and appreciate the general differences in structural arrangement between the microchambers and the microcapsules.

Microchambers can be converted into microcapsules, and the present invention provides several methods of achieving this, discussed further below.

The microcapsules may have a largest dimension of up to 50 microns. For example, in the case of spherical microcapsules (or substantially spherical microcapsules), the microcapsules may have a diameter of up to about 50 microns. For microcapsules of other shapes, the largest dimension refers to the straight-line distance between the two points of the microcapsules that are furthest from each other. Preferably, the microcapsules are not spherical. Instead, the microcapsules preferably have at least one flat side. In some embodiments, the microcapsules have at least 2 opposing flat sides (given the method for their manufacture requires pressing together templates). The 2 opposing flat sides may be substantially parallel.

In some embodiments, the microcapsules may be polyhedral or substantially polyhedral. In some embodiments, the microcapsules may be polyhedral or substantially polyhedral with up to 6 sides. For example, in some embodiments, the microcapsules may be cuboidal, substantially cuboidal, frustopyramidal (truncated pyramid shape) or substantially frustopyramidal in shape. In embodiments in which the microcapsules are polyhedral or substantially polyhedral with up to 6 sides, at least one of the sides may be flat, and optionally the microcapsule comprising 2 opposing flat sides that are substantially parallel with one another.

For microchambers, the dimensions refer to the size of the wells in the stamp used to form the microchambers. The microchambers may similarly have a largest dimension of up to 50 microns. For example, the straight-line distance between the two points of a given well in the first stamp that are furthest from each other may be up to 50 microns. The microchambers in the film may have varying periodicity, as determined by the pattern of wells in the first stamp. The periodicity of the microchambers or wells may refer to the distance between the centre of adjacent microchambers or wells. The periodicity may be optimised by the skilled person, for example to alter the amount of drug in the final composition. The periodicity can be, for example, from about 2 microns to about 100 microns, or from about 5 microns to about 100 microns. In some embodiments, the distance between the wells (and hence the distance between adjacent microchambers) may also be optimised by the skilled person. In some embodiments, the distance between the wells may be from about 5 microns, for example from about 5 to about 100 microns. The precise dimensions are, however, not crucial. Preferably, the microchambers are not spherical. Instead, the microchambers preferably have at least one flat side. In some embodiments, the microchambers have at least 2 opposing flat sides (given the method for their manufacture requires pressing together templates). The 2 opposing flat sides may be substantially parallel.

In some embodiments, the microchambers may be polyhedral or substantially polyhedral. In some embodiments, the microchambers may be polyhedral or substantially polyhedral with up to 6 sides. For example, in some embodiments, the microchambers may be cuboidal, substantially cuboidal, frustopyramidal (truncated pyramid shape) or substantially frustopyramidal in shape. In embodiments in which the microchambers are polyhedral or substantially polyhedral with up to 6 sides, at least one of the sides may be flat, and optionally the microchamber comprising 2 opposing flat sides that are substantially parallel with one another.

The microchambers and microcapsules are uniform in size. In some embodiments, at least 90% of the microchambers or microcapsules have a size (i.e. volume) that is within 10% of the average (mean) size (i.e. volume) of the microchambers or microcapsules in the composition. Hence the microchambers and microcapsules have a narrow size distribution.

The size of the core will be reduced according to the thickness of the polymer shell. Generally, the thickness of the shell may be at least about 0.05 microns. In some embodiments, the thickness of the shell may be at least about 0.1 microns. In some embodiments, the thickness of the shell may be from about 0.05 to about 2 microns (for example from about 0.1 to about 2 microns or from about 0.1 to about 0.5 microns). The thickness of the shell may vary depending on the shape of the microcapsule or microchamber. The thickness of the shall may also not be uniform (i.e. the shell may have a non-uniform thickness). Indeed, given the method of manufacture of the microparticles and microcapsules, the shell is likely to have a non-uniform thickness. In some embodiments, at least about 10%, at least about 20%, at least about 30%, at least about 40% or at least 50% of the shell has a thickness of from about 0.05 to about 2 microns (for example from about 0.1 to about 2 microns or from about 0.1 to about 0.5 microns). In other parts, the shell may be thicker than at least 2 microns. The thickness of the shell may influence the release provided by the sustained release composition.

The size of the core will be smaller than the size of the microparticles or microchambers.

In preferred embodiments, the shell completely encapsulates the core. This complete encapsulation, which is optimised when using the methods of the present invention, helps to provide sustained release compositions having very sustained release profiles, as demonstrated in the examples. The shell may completely encapsulate the core with a minimum shell thickness of at least about 0.05 microns, for example at least about 0.1 microns.

In some embodiments, the core may comprise air. In some embodiments, the core may consist of the crystalline drug and air (for example, although not exclusively, in the case of the "no heat" methods of the present invention).

In some embodiments, the core may consist of the crystalline drug only (for example, although not exclusively, in the case of the "heat and pressure" methods of the present invention). However, other components may be present. For example, in some embodiments, the core may consist of the crystalline drug and optionally polymer (for example, although not exclusively, in the case of the "heat and pressure" methods of the present invention where the polymer of the shell mixes with the crystalline drug when it melts during the manufacturing process). Therefore, in some embodiments, (for example those relating to the "heat and pressure" methods of the present invention and the compositions made therefrom), the drug may be completely or partially mixed with the polymer shell, such that the core comprises both drug and polymer. However, the shell still fully encapsulates the core, providing a shell that is at least 0.05 microns thick (for example at least 0.1 microns thick). The shell consists of the polymer and does not comprise any drug. In some embodiments, in particular embodiments relating to the heat and pressure method, the core does not comprise air. However, in other embodiments, the core may further comprise air. Thus, for example in some embodiments, the core may consist of the crystalline drug and polymer and optionally air.

The size of the drug crystals may be varied by the skilled person. Smaller drug crystals may allow a larger amount of drug to be loaded into the microcapsules.

The compositions of the invention may have varying ratios of polymer to crystalline drug. In some embodiments, the composition comprises at least about 10%, at least about 20%, at least about 30%, at least about 40% or at least about 50% crystalline drug by weight. In some embodiments, the composition comprises from about 50% to about 70% crystalline drug by weight (for example, but not limited to, embodiments in which the compositions are made according to the "no heat" method. Compositions made by other methods may have a lower amount of encapsulated drug).

The compositions of the invention can be used to microencapsulate a range of different drugs. Generally, the drug will have a solubility that is less than 1mg/ml in water at 25 °C. The solubility may be determined at 1 atmospheric pressure. Such solubility thresholds may be particularly relevant when the drug is a corticosteroid. In embodiments in which the drug is a protein, solubility may be higher than this.

The drug may alternatively or additionally have a melting point above the melting point of the polymer (in particular in embodiments involving the "heat and pressure" method of the invention). For example, in some embodiments the drug may have a melting point above 200 °C.

In some embodiments, the drug may be susceptible to denaturing upon heating. For example, in embodiments where the drug is a protein, methods of the invention can be used to encapsulate the drug without a heating step, yet still be able to provide compositions with extended sustained release profiles.

The drugs encapsulated in the compositions and by the methods of the invention are crystalline. Therefore, the drugs are not liquid and are not in solution. Instead, dry drug is loaded into the wells. This allows the encapsulation of any drugs that can be provided in dried form.

The present invention is particularly relevant to corticosteroids and proteins.

In some embodiments, the corticosteroid is selected from the group consisting of dexamethasone, prednisolone, betamethasone, prednisone, methylprednisolone, budesonide, hydrocortisone, triamcinolone and fludrocortisone. In some embodiments, the corticosteroid is dexamethasone or prednisolone. In specific embodiments, the corticosteroid is dexamethasone.

In some embodiments, the drug is a protein. For example, in some embodiments, the protein is a growth factor, an enzyme, a cytokine, a chemokine or a biological therapeutic (for example a monoclonal antibody).

The shell component of the sustained release compositions is biodegradable. In some embodiments, the shell has a melting point of at least about 50°C In some embodiments, the shell has a melting point of from about 50°C to about 200°C.

The polymer composition (e.g. polymer or polymer blend) used to form the shell is generally insoluble in water.

In some embodiments, the polymer used for the shell has an average molecular weight of up to 200 kDa, for example up to 100 kDa. In some embodiments the polymer has an average molecular weight of from about 1 kDa to about 200 kDa, or from about 30 kDa to about 200 kDa, or from about 40 kDa to about 100 kDa, for example of from about 50 kDa to about 70 kDa.

The shell can consist of a single type of polymer or the shell may comprise or consist of a mixture or blend of polymers. For example, in some embodiments, the shell comprises a polymer selected from the group consisting of PLA, PLGA, PLA/PLGA and PCL, or a blend thereof.

In some embodiments, the shell comprises:
a) Polylactic acid (PLA) homopolymer
b) PLA/PLGA copolymer (i.e. a blend of the homopolymer PLA and the copolymer PLGA)
c) PLGA copolymer
d) PLA and polycaprolactone (PCL) (i.e. a polymer blend of the homopolymer PLA and the homopolymer PCL)
e) PLGA and PCL (i.e. a polymer blend of the copolymer PLGA and the homopolymer PCL); or
f) PLA/PLGA and PCL (i.e. a polymer blend of the homopolymer PLA, the copolymer PLGA and the homopolymer PCL).

In some embodiments, the shell consists of:
a) Polylactic acid (PLA) homopolymer
b) PLA/PLGA copolymer (i.e. a blend of the homopolymer PLA and the copolymer PLGA)
c) PLGA copolymer
d) PLA and polycaprolactone (PCL) (i.e. a polymer blend of the homopolymer PLA and the homopolymer PCL)
e) PLGA and PCL (i.e. a polymer blend of the copolymer PLGA and the homopolymer PCL); or
f) PLA/PLGA and PCL (i.e. a polymer blend of the homopolymer PLA, the copolymer PLGA and the homopolymer PCL).

In some embodiments, the shell consists of PLA (for example a PLA homopolymer).

In embodiments wherein the shell comprises or consists of PLA and PCL, the shell may have more PLA than PCL by weight. In some embodiments, the shell comprises or consists of PLA and PCL and the shell comprises a PLA:PCL ratio of at least 2:1 by weight.

In some embodiments, the polymeric shell does not comprise PVA.

The compositions of the invention may further comprise one or more pharmaceutically acceptable excipients or diluents. Suitable excipients may include, for example, phosphate buffered saline (PBS).

The compositions of the invention are particularly suited to being injectable or implantable. For example, in some embodiments, in particular those embodiments comprising microcapsules, the compositions may be injectable. Injectable compositions may comprise a suspension of microcapsules of the invention, for example in PBS.

The sustained-release compositions of the invention have advantageous release profiles. The compositions are an improvement over the prior art, for example because they do not have a burst release from inside the microchambers or microwells (i.e. a high release of encapsulated drug) shortly after administration or implantation. Therefore, in some embodiments, the drug release profile of sustained-release composition does not comprise a burst release. A burst release may be defined as a bulk release, for example of up to 1% of the loaded drug (or up to 5% of the loaded drug), within 1 day of administration or implantation. Instead, the drug is released steadily over a long period.

In some embodiments the sustained-release compositions of the invention have extended release profiles that release the drug very slowly. For example, in some embodiments, less than 50% of the drug by weight is released after the microchambers or microcapsules are in aqueous solution for 24 weeks. In some embodiments, the release profile may be measured according to the following protocol:
a) optionally immersing the microchambers or microcapsules in sterile water for 3 months;
b) immersing the microchambers or microcapsules in a cell culture medium;
c) incubating the immersed microchambers or microcapsules at 37°C for 1 week;
d) measuring the amount of drug that is released from the microchambers or microcapsules into the cell culture medium;
e) completely replacing the cell culture medium;
f) repeating steps (c) to (e) for 24 weeks; (i.e. these steps are performed a total of 24 times)
g) determining the cumulative amount of drug released from the microchambers or microcapsules into the cell culture medium after 24 weeks;
h) using the amount determined in step (g), calculating the amount of drug that was released into the cell culture medium as a percentage of the total drug present in the microchambers or microcapsules at the start of step (b).

The total amount of drug present in the microchambers or microcapsules at the start of step (b) may be determined by causing the microchambers or microcapsules to release all remaining encapsulated drug, for example by sonication. The cell culture medium is maintained at 37°C for the duration of the 24 week period.

In some embodiments, the release of drug may be even slower. For example, in some embodiments, less than 1% of the drug by weight, for examples less than 0.1% by weight may be released after 12 weeks. In some embodiments, the release profile may be measured according to the following protocol:
a) optionally immersing the microchambers or microcapsules in sterile water for 2 days;
b) replacing the sterile water to immerse the microchambers or microcapsules in 1ml of sterile water;
c) incubating the immersed microchambers or microcapsules for 1 week at 37 °C
d) removing and replacing 300 µl of water and measuring the amount of drug released from the microchambers or microcapsules during the incubation period;
e) repeating steps (c) and (d) for 5 weeks; (i.e. these steps are performed a total of 5 times)
f) immersing the microchambers or microcapsules in cell culture medium;
g) incubating the immersed microchambers or microcapsules for 1 week at 37 °C;
h) measuring the amount of drug released into the cell culture medium;
i) completely replacing the cell culture medium;
j) repeating steps (g) and (i) for 7 weeks; (i. e. these steps are performed a total of 7 times)
k) determining the cumulative amount of drug released from the microchambers or microcapsules over the 12 weeks from step (b) to step (j);
l) using the amount determined in step (k), calculating the amount of drug that was released from the microchambers or microcapsules as a percentage of the total drug present in the microchambers or microcapsules at the start of step (b).

The total amount of drug present in the microchambers or microcapsules at the start of step (b) may be determined by causing the microchambers or microcapsules to release all remaining encapsulated drug, for example by sonication. The cell culture medium is maintained at 37°C for the duration of the 24 week period. Step (d) above will require using the concentration or amount of drug on the 300 µl to determine the total amount of drug released from the microchambers or microcapsules during the incubation period.

The cell culture medium can be, for example, a basal mammalian cell culture medium. Such a medium may be suitable for the culture of many different mammalian cell types, such as primary fibroblasts, neurons, glial cells, HUVECs, and smooth muscle cells, as well as cell lines such as HeLa, 293, Cos-7, and PC-12. An example medium is Gibco Dulbecco's Modified Eagle Medium (DMEM). Such a cell culture medium can have a composition as show in Table 1:

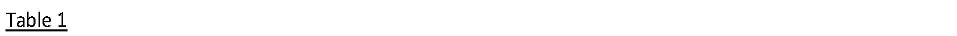

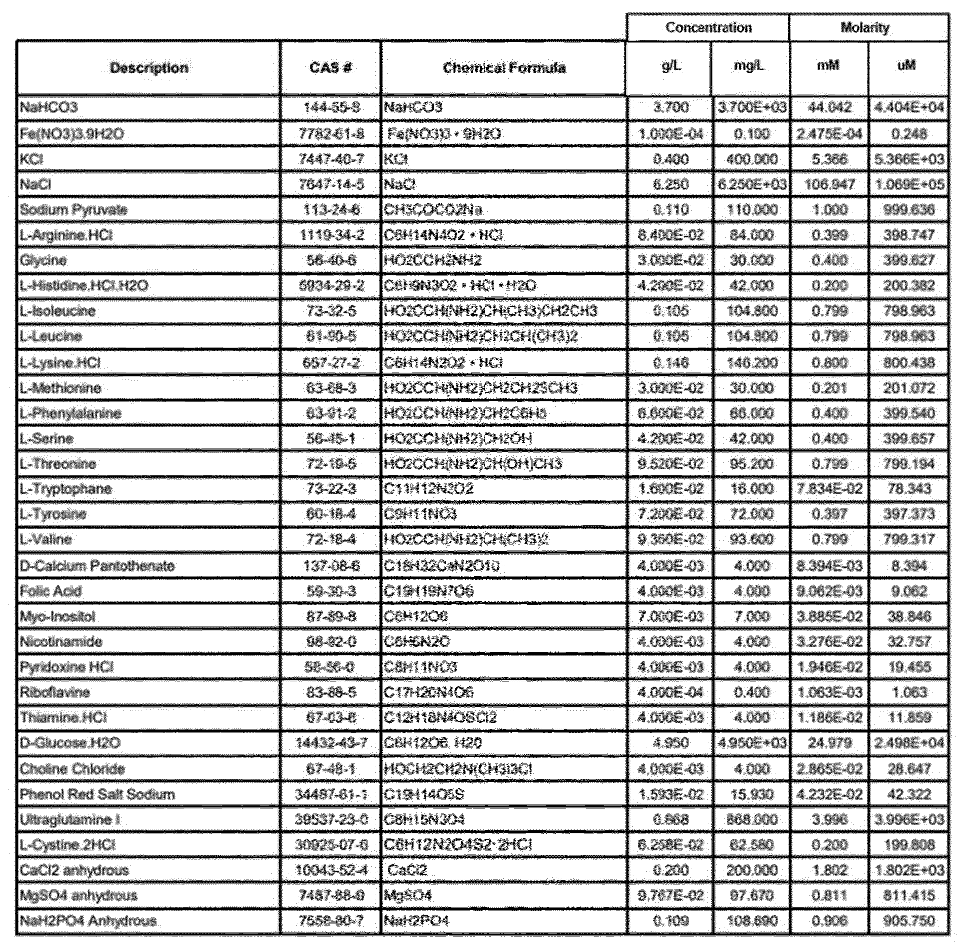

Release of the encapsulated drug may be triggered. For example, exposure of the microchambers or microcapsules to ultrasound may cause the encapsulated drug to be released. This may have use in certain contexts, for example in the case of triggering a burst of drug release at specific times, for example after implantation into or administration to a patient.

In some embodiments of the invention, the compositions of the invention comprise:
a) a sustained-release composition comprising a plurality of microcapsules, wherein:
   i. the microcapsules comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell comprises of polylactic acid (PLA), wherein the shell has a thickness of at least about 0.05 microns and the shell has a melting point of from about 50°C to about 200°C;
   iv. the shell completely encapsulates the core;
b) a sustained-release composition comprising a plurality of microcapsules, wherein:
   i. the microcapsules comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell consists of polylactic acid (PLA), wherein the shell has a thickness of at least about 0.05 microns;
   iv. the shell completely encapsulates the core;
c) a sustained-release composition comprising a plurality of microcapsules, wherein:
   i. the microcapsules comprise a core and a biodegradable shell;
   ii. the core consists of crystalline dexamethasone and optionally air;
   iii. the shell comprises of polylactic acid (PLA), wherein the shell has a thickness of at least about 0.05 microns and the shell has a melting point of from about 50°C to about 200°C;
   iv. the shell completely encapsulates the core;
d) a sustained-release composition comprising a plurality of microcapsules, wherein:
   i. the microcapsules comprise a core and a biodegradable shell;
   ii. the core consists of crystalline dexamethasone and optionally air;
   iii. the shell consists of polylactic acid (PLA), wherein the shell has a thickness of at least about 0.05 microns;
   iv. the shell completely encapsulates the core;
e) a sustained-release composition comprising a film of microchambers, wherein:
   i. the microchambers comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell comprises of polylactic acid (PLA), wherein the shell has a thickness of at least about 0.05 microns and the shell has a melting point of from about 50°C to about 200°C;
   iv. the shell completely encapsulates the core;
f) a sustained-release composition comprising a film of microchambers, wherein:
   i. the microchambers comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell consists of polylactic acid (PLA), wherein the shell has a thickness of at least about 0.05 microns;
   iv. the shell completely encapsulates the core;
g) a sustained-release composition comprising a film of microchambers, wherein:
   i. the microchambers comprise a core and a biodegradable shell;
   ii. the core consists of crystalline dexamethasone and optionally air;
   iii. the shell comprises of polylactic acid (PLA), wherein the shell has a thickness at least about 0.05 microns and the shell has a melting point of from about 50°C to about 200°C;
   iv. the shell completely encapsulates the core; or
h) a sustained-release composition comprising a film of microchambers, wherein:
   i. the microchambers comprise a core and a biodegradable shell;
   ii. the core consists of crystalline dexamethasone and optionally air;
   iii. the shell consists of polylactic acid (PLA), wherein the shell has a thickness of at least about 0.05 microns;
   iv. the shell completely encapsulates the core.
i) a sustained-release composition comprising a plurality of microcapsules, wherein:
   i. the microcapsules comprise a core and a biodegradable shell;
   ii. the shell comprises of polylactic acid (PLA), wherein the shell has a thickness of at least about 0.05 microns and the shell has a melting point of from about 50°C to about 200°C;
   iii. the core consists of a crystalline corticosteroid and optionally polymer of the biodegradable shell, and further optionally air, and the corticosteroid has a solubility of less than 1mg/ml in water at 25 °C and a melting point of at least 200°C;
   iv. the shell completely encapsulates the core;
j) a sustained-release composition comprising a plurality of microcapsules, wherein:
   i. the microcapsules comprise a core and a biodegradable shell;
   ii. the shell consists of polylactic acid (PLA), wherein the shell has a thickness of at least about 0.05 microns;
   iii. the core consists of a crystalline corticosteroid and optionally polymer of the biodegradable shell, and further optionally air, and the corticosteroid has a solubility of less than 1mg/ml in water at 25 °C and a melting point of at least 200°C;
   iv. the shell completely encapsulates the core;
k) a sustained-release composition comprising a plurality of microcapsules, wherein:
   i. the microcapsules comprise a core and a biodegradable shell;
   ii. the shell comprises of polylactic acid (PLA), wherein the shell has a thickness of at least about 0.05 microns and the shell has a melting point of from about 50°C to about 200°C;
   iii. the core consists of crystalline dexamethasone and optionally polymer of the biodegradable shell, and further optionally air;
   iv. the shell completely encapsulates the core;
l) a sustained-release composition comprising a plurality of microcapsules, wherein:
   i. the microcapsules comprise a core and a biodegradable shell;
   ii. the shell consists of polylactic acid (PLA), wherein the shell has a thickness of at least about 0.05 microns;
   iii. the core consists of crystalline dexamethasone and optionally polymer of the biodegradable shell, and further optionally air;
   iv. the shell completely encapsulates the core;
m) a sustained-release composition comprising a film of microchambers, wherein:
   i. the microchambers comprise a core and a biodegradable shell;
   ii. the shell comprises of polylactic acid (PLA), wherein the shell has a thickness of at least about 0.05 microns and the shell has a melting point of from about 50°C to about 200°C;
   iii. the core consists of a crystalline corticosteroid and optionally polymer of the biodegradable shell, and further optionally air, and the corticosteroid has a solubility of less than 1mg/ml in water at 25 °C and a melting point of at least 200°C;
   iv. the shell completely encapsulates the core;
n) a sustained-release composition comprising a film of microchambers, wherein:
   i. the microchambers comprise a core and a biodegradable shell;
   ii. the shell consists of polylactic acid (PLA), wherein the shell has a thickness of at least about 0.05 microns;
   iii. the core consists of a crystalline corticosteroid and optionally polymer of the biodegradable shell, and further optionally air, and the corticosteroid has a solubility of less than 1mg/ml in water at 25 °C and a melting point of at least 200°C;
   iv. the shell completely encapsulates the core;
o) a sustained-release composition comprising a film of microchambers, wherein:
   i. the microchambers comprise a core and a biodegradable shell;
   ii. the shell comprises of polylactic acid (PLA), wherein the shell has a thickness at least about 0.05 microns and the shell has a melting point of from about 50°C to about 200°C;
   iii. the core consists of crystalline dexamethasone and optionally polymer of the biodegradable shell, and further optionally air;
   iv. the shell completely encapsulates the core; or
p) a sustained-release composition comprising a film of microchambers, wherein:
   i. the microchambers comprise a core and a biodegradable shell;
   ii. the shell consists of polylactic acid (PLA), wherein the shell has a thickness of at least about 0.05 microns;
   iii. the core consists of crystalline dexamethasone and optionally polymer of the biodegradable shell, and further optionally air;
   iv. the shell completely encapsulates the core.
q) a sustained-release composition comprising a plurality of microcapsules, wherein:
   i. the microcapsules comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell comprises of polylactic acid (PLA), wherein the shell has a thickness of from about 0.05 microns to about 2 microns and the shell has a melting point of from about 50°C to about 200°C;
   iv. the shell completely encapsulates the core;
r) a sustained-release composition comprising a plurality of microcapsules, wherein:
   i. the microcapsules comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell consists of polylactic acid (PLA), wherein the shell has a thickness of from about 0.05 microns to about 2 microns;
   iv. the shell completely encapsulates the core;
s) a sustained-release composition comprising a plurality of microcapsules, wherein:
   i. the microcapsules comprise a core and a biodegradable shell;
   ii. the core consists of crystalline dexamethasone and optionally air;
   iii. the shell comprises of polylactic acid (PLA), wherein the shell has a thickness of from about 0.05 microns to about 2 microns and the shell has a melting point of from about 50°C to about 200°C;
   iv. the shell completely encapsulates the core;
t) a sustained-release composition comprising a plurality of microcapsules, wherein:
   i. the microcapsules comprise a core and a biodegradable shell;
   ii. the core consists of crystalline dexamethasone and optionally air;
   iii. the shell consists of polylactic acid (PLA), wherein the shell has a thickness of from about 0.05 microns to about 2 microns;
   iv. the shell completely encapsulates the core;
u) a sustained-release composition comprising a film of microchambers, wherein:
   i. the microchambers comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell comprises of polylactic acid (PLA), wherein the shell has a thickness of from about 0.05 microns to about 2 microns and the shell has a melting point of from about 50°C to about 200°C;
   iv. the shell completely encapsulates the core;
v) a sustained-release composition comprising a film of microchambers, wherein:
   i. the microchambers comprise a core and a biodegradable shell;
   ii. the core consists of a crystalline corticosteroid and optionally air, and the corticosteroid has a solubility of less than 1mg/ml in water at 25 °C and a melting point of at least 200°C;
   iii. the shell consists of polylactic acid (PLA), wherein the shell has a thickness of from about 0.05 microns to about 2 microns;
   iv. the shell completely encapsulates the core;
w) a sustained-release composition comprising a film of microchambers, wherein:
   i. the microchambers comprise a core and a biodegradable shell;
   ii. the core consists of crystalline dexamethasone and optionally air;
   iii. the shell comprises of polylactic acid (PLA), wherein the shell has a thickness of from about 0.05 microns to about 2 microns and the shell has a melting point of from about 50°C to about 200°C;
   iv. the shell completely encapsulates the core; or
x) a sustained-release composition comprising a film of microchambers, wherein:
   i. the microchambers comprise a core and a biodegradable shell;
   ii. the core consists of crystalline dexamethasone and optionally air;
   iii. the shell consists of polylactic acid (PLA), wherein the shell has a thickness of from about 0.05 microns to about 2 microns;
   iv. the shell completely encapsulates the core.

Other microcapsules and microchambers are within the scope of the present invention.

### Methods of preparing the sustained release compositions

The present invention provides methods for making the compositions of the inventions, and compositions obtained or obtainable by such compositions. In particular, the present invention provides methods of making microchambers, and two different methods of making microcapsules. The two methods of making microcapsules are referred to herein as the "no heat method" and the "heat and pressure method". Both methods provide compositions with advantageous properties. The methods of the invention may clearly be combined with the more detailed embodiments of the compositions of the invention, since the methods of the invention are used to produce said compositions.

In some embodiments, the methods are methods for micro-encapsulating a crystalline drug, comprising:
a. providing a first stamp comprising a plurality of microwells, wherein the microwells of the first stamp are coated with a polymer composition comprising polylactic acid (PLA);
b. loading crystalline drug into the coated microwells;
c. providing a second stamp, wherein the second stamp is planar and is coated on at least one side with the polymer composition comprising polylactic acid (PLA); and
d. pressing the coated sides of the first and second stamps together to encapsulate the crystalline drug in the wells with the polymer composition to form a film of microchambers.

The methods may comprise a step of coating the first and/or second stamps with the polymer composition. The polymer layer may have a thickness of at least about 0.05 microns (for example at least about 0.1 microns). For example, the polymer layer may have a thickness of from about 0.05 to about 2 microns, and the thickness of the layer will determine the thickness of some of the walls of the resulting microchambers and microcapsules. Coating may take place by any suitable method, for example dip coating. The polymer composition may be dried prior to loading of the drug.

In some embodiments, the methods comprise a step of providing the first stamp comprising a plurality of microwells, and coating the microwells with a solution comprising the polymer composition, and/or a step of providing the second stamp and coating at least one side with an solution comprising the polymer composition. The solution of the polymer composition may have a concentration of from about 0.1% to about 25%, or from about 0.1% to about 5%, or from about 0.5 to about 2%, or about 1%. The concentrations are provided as weight/weight %. For example, 1% solution means there is 10mg of polymer dissolved in 1g of solution.

In some methods, in particular those comprising the use of a solution of polymer composition, the methods may comprise a step of evaporating the solvent of the polymer solution, to provide a solid polymer composition.

The stamps used in the methods of the invention may be of any suitable material. For example, the stamps may be silicone stamps or glass stamps. In some embodiments the stamps are polydimethylsiloxane (PDMS) stamps or poly(methyl methacrylate) (PMMM) stamps. Generally, the stamps will have a higher melting point than the drug or polymer composition, especially in methods comprising the use of heat.

The second stamp, although generally planar, may comprise a series of indentations or projections that align with the wells in the first stamp, for example to help determine particular shape for the microchambers or microcapsules. The second stamp can also be flat. The second stamp may also be a glass slide.

The methods may comprise a step of removing the film of microchambers from the first stamp, after the step of pressing. Prior to the removal step, the method comprises a step of separating the first and second stamps.

This provides a film of microchambers that can be used for a number of different purposes, for example those discussed herein such as an implantable device or affixing to an implantable device to form a drug-eluting implantable device.

Since the microchambers may be washed before and/or after removal from the first stamp to remove excess crystalline drug.

Removal of the film of microchambers from the stamps may be achieved in a number of ways. Given the high tensile strength of the polymer composition, the film of microchambers can be removed from the stamp by hand.

Alternatively, in some embodiments, the step of removing the film of microchambers from the first stamp comprises:
a. providing a planar substrate coated with a soluble adhesive;
b. pressing the planar substrate onto the first stamp to adhere the film of microchambers to the slide;
c. removing the film of microchambers from the microwells of the first stamp by separating the planar substrate and the first stamp.

The planar substrate can be any suitable planar substrate, for example a glass slide. The soluble adhesive can be gelatin or PVA.

The method may further comprise a step of dissolving the soluble adhesive to provide a detached film of microchambers. The film of microchambers may be washed to remove excess drug that was not encapsulated by the process.

Loading of the drug into the microwells may be achieved by loading crystalline drug directly into the wells. Alternatively, loading may be achieved by, for example, adding a solution (for example an aqueous solution) of drug to the microwells and allowing the solute to evaporate to leave a crystalline drug deposit in the wells. Such deposition methods may be particularly relevant when the drug is a protein. However, in some embodiments, loading of the drug into the microwells may be achieved without using drug dissolved in a solvent and instead the drug is loaded directly into the microwells in crystalline form. Such embodiments may be beneficial since they allow the method to be performed without having to use any solvents to dissolve the drug prior to loading. This also means the solubility of the drug is not a factor, since it does not need to be dissolved to enable it to be loaded into the microwells.

Prior to pressing the stamps together, there may be a step of removing excess drug. For example, drug crystals in the spaces between the wells in the first stamp may be removed prior to pressing the stamps together.

More specific methods are provided for the production of microcapsules. These methods are the "no heat" method and the "heat and pressure method".

The no heat method comprises pressing the coated sides of the first and second stamps together without heat. The step of pressing the coated sides of the first and second stamps together may be carried out at at a pressure of up to about 0.25 MPa (for example up to about 0.1 MPa, such as from about 0.01 MPa up to about 0.25 MPa or from about 0.01 MPa up to about 0.1MPa). Generally in these methods, the step of pressing the stamps together is performed at a temperature less than the melting point of the polymer composition. In some embodiments, the step of pressing the stamps together is performed at a temperature of less than 50°C (for example at room temperature, such as between 15°C and 25°C). Even though the polymer composition is not heated, the pressing of the stamps together causes the layer of polymer composition on the first stamp to adhere to (and combine with) the layer of polymer composition on the second stamp. Thus, the stamps are pressed together at a pressure sufficient to fuse the layers of polymer together and encapsulate the drug.

Pressing of the stamps together may take place for up to about 5 minutes.

In some embodiments, the method does not comprise a step of heating the polymer composition.

Once the stamps are pressed together, they may be separated to facilitate removal of the microencapsulated drug. The "no heat" methods of the invention include a step of removing excess polymer composition from the first stamp after separating the first and second stamps. This may be achieved by any suitable mechanical action or means, for example, scraping the excess polymer away. The step of removing excess polymer composition separates the microchambers into separate microcapsules in the wells of the first stamp. For example, the film of microchambers comprises a plurality of chambers (formed by the polymer shells), the plurality of chambers existing in a planar continuum of polymer composition. In addition to the plurality of chambers, the film further comprises inter-chamber zones of polymer that attach adjacent chambers together. The inter-chamber zones of polymer connect the chambers together to form a continuous planar layer of the polymer film that joins the chambers together in a single sheet. The step of removing excess polymer composition removes some or all of the polymer composition present between adjacent chambers (i.e. the microcapsules are separated by removal of some or all of the inter-chamber zones of polymer), thus separating the chambers to provide a plurality of microcapsules. The step of removing the excess polymer to separate adjacent chambers therefore comprises breaking the connections between adjacent chambers to provide the microcapsules. The plurality of separated microcapsules so formed are inside the wells of the first plate, and are later removed from the stamp, for example for formulation into a pharmaceutical composition.

After the microchambers are separated into microcapsules, the method may further comprise a step of removing the microcapsules from the wells of the first stamp. This may be achieved by any suitable means. For example, in some embodiments, and as discussed above for the removal of a film of microchambers, the step of removing the microcapsules from the stamp may comprise:
a. providing a planar substrate coated with a soluble adhesive;
b. pressing the planar substrate onto the first stamp to adhere the microcapsules to the slide;
c. removing the microcapsules from the microwells of the first stamp by separating the planar substrate and the first stamp.

The planar substrate can be any suitable planar substrate, for example a glass slide. The soluble adhesive can be gelatin or PVA. The method may further comprise a step of dissolving the soluble adhesive to separate the microcapsules from the planar substrate. The microcapsules may be washed to remove excess drug that was not encapsulated by the process.

The "no heat" methods of the invention are particularly suited to encapsulation of drugs that are susceptible to denaturation when heated (for example proteins) or to those with low melting points. However, the methods can be used for microencapsulation of other drugs, such as corticosteroid.

Alternatively, there is provided the "heat and pressure" method for providing microcapsules. Such methods comprise pressing the stamps together and simultaneously heating the polymer composition. The polymer composition may be heated to a temperature equal to or greater than the melting point of the polymer composition. However, it may not be necessary to reach the melting point of the polymer composition. In some embodiments, the polymer composition may be heated to a temperature that is greater than a temperature that is 20°C below the melting point of the polymer composition (so for example, if the melting point of the polymer composition is 100°C, the polymer composition is heated to above 80°C). In some embodiments, the polymer composition may be heated to a temperature higher than a temperature that is 10°C below the melting point.

For example, in some embodiments, the methods comprise heating the polymer composition to a temperature of at least about 50 °C. In some embodiments, the methods comprise heating the polymer composition to a temperature of from about 50 °C to about 200 °C, from about 100 °C to about 200 °C, for example from about 130 °C to about 170 °C, or from about 140 °C to about 160 °C.

The pressures applied in these methods can vary. In some embodiments, the method comprises pressing the coated sides of the first and second stamps together at a pressure of at least about 0.1 MPa, at least about 0.2 MPa, at least about 0.3 MPa, at least about 0.4 MPa or at least about 0.5 MPa.

The step of pressing the stamps together while heating separates the microchambers into separate microcapsules. It is hypothesised this is achieved as a result of the polymer composition melting and molding to the shape of the wells in the first stamp. The microchambers separate into microcapsules as the polymer composition forming the shell of each microchamber separates from adjacent microchambers (i.e. the connections between adjacent microchambers are broken), upon application of the heat and pressure.

The step of heating may occur for up to about 30 minutes, for example up to about 15 minutes. Preferably, the heating step comprising heating for no more than 5 minutes.

Once the microchambers are separated into microcapsules, the first and second stamps may be separated to facilitate removal of the microcapsules from the wells of the first stamp. (The separation of the two stamps may occur after the stamps and polymer composition have been allowed to cool to room temperature). The method may therefore further comprise a step of removing the microcapsules from the wells of the first stamp. This may be achieved by any suitable means. For example, in some embodiments, and as discussed above for the removal of a film of microchambers, the step of removing the microcapsules from the stamp may comprise:
a. providing a planar substrate coated with a soluble adhesive;
b. pressing the planar substrate onto the first stamp to adhere the microcapsules to the slide;
c. removing the microcapsules from the microwells of the first stamp by separating the planar substrate and the first stamp.

Between steps (b) and (c) there may be a step of cooling the planar substrate and first stamp, for example to a temperature that is less than °C. A step of cooling may take place to solidify the soluble adhesive.

Step (c) may include one or more washing steps to remove excess of adhesive. The washing step may be performed according to any suitable means, for example by suspending the microcapsules in aqueous solution and centrifuging the suspension, replacing the supernatant, and resuspending the microcapsules.

The planar substrate can be any suitable planar substrate, for example a glass slide. The soluble adhesive can be gelatin or PVA. The method may further comprise a step of dissolving the soluble adhesive to separate the microcapsules from the planar substrate. The microcapsules may be washed to remove excess drug that was not encapsulated by the process.

Once the microchambers or microcapsules are provided, they may be further processed. For example, in some embodiments the microchambers or microcapsules may be dried (for example freeze dried). The microchambers or microcapsules may also be formulated, for example with one or more pharmaceutically acceptable excipients or diluents. The microchambers or microcapsules may be formulated into an injectable pharmaceutical composition.

### Other methods and uses of the invention

The present invention provides a number of other methods and uses. For example, there is provided a sustained-release composition comprising a plurality of microcapsules obtained or obtainable by the methods of the invention. There is also provided a sustained-release composition comprising a film of microchambers obtained or obtainable by the methods of the invention.

The methods of the invention also relate to methods of treatment. For example, there is provided a method of treating a disease or disorder, comprising administering a sustained-release composition of the invention to a subject in need therefore. The composition is provided to the patient in a therapeutically effective amount.

The invention also provides use of a plurality of microcapsules of the invention in the manufacture of a sustained-release medicament to treat a disease or disorder. The sustained-release medicament may be injectable. Similarly, the invention provides a sustained-release composition according to the invention (for example an injectable composition) for use in the treatment of a disease or disorder.

The disease or disorder to be treated may be any disease or disorder than can be treated using the microencapsulated drug. For example, the disease or disorder is an inflammatory disease or disorder.

Certain diseases or disorders may be particularly relevant to corticosteroid drugs. For example, the disease or disorder may be pain, inflammation, arthritis (for example osteoarthritis or rheumatoid arthritis), inflammatory bowel diseases (IBD), Crohn's disease, ulcerative colitis, multiple sclerosis, polymyalgia rheumatica, asthma, allergies, chronic obstructive pulmonary disease (COPD), croup or sciatica.

In some embodiments, the disease or disorder is arthritis, for example osteoarthritis or rheumatoid arthritis.

### Drug-eluting implantable medical devices

The compositions of the invention are particularly suited to drug-eluting implantable medical devices, since the compositions provide controlled release of the encapsulated drug over a very long period. There is therefore provided a drug-eluting implantable medical device comprising or consisting of a film of microchambers of the invention. The present invention also provides a method of preparing the drug-eluting implantable medical device, comprising providing an implantable medical device and affixing a film of microchambers of the invention to the implantable medical device.

The implantable medical device can be any suitable implantable medical device. For example, in some embodiments, the implantable medical device is a stent or a replacement joint. The film of microchambers may itself be used as an implantable medical device. Alternatively, the film of microchambers may by adhered to or incorporated into a scaffold, in particular a biodegradable scaffold, and used as an implantable medical device in that format.

The invention provides methods of using the implantable medical devices. For example, there is provided a method of surgery, comprising implanting a film of microchambers according to invention, or a drug-eluting implantable medical device of the invention, into a patient (for example affixing a film of microchambers according to invention, or a drug-eluting implantable medical device of the invention, to the patient). The film of microchambers or drug-eluting implantable medical device may be affixed to an intracorporeal surface of the patient. For example, the film of microchambers or drug-eluting implantable medical device is affixed to an internal organ, a body cavity, or a bone. The body cavity may be the peritoneal cavity.

Methods of surgery may include additional steps. For example, the methods may comprise a step of making an incision into a patient, implanting a film of microchambers or medical device of the invention into the patient, and closing the incision.

Preferred features for the second and subsequent aspects of the invention are as for the first aspect mutatis mutandis.

The present invention will now be further described by way of illustration only with reference to the following Examples, which are not to be construed as being limiting on the invention.

### EXAMPLES

### Example 1: Preparation of biodegradable crystalline drug loaded microchamber array films

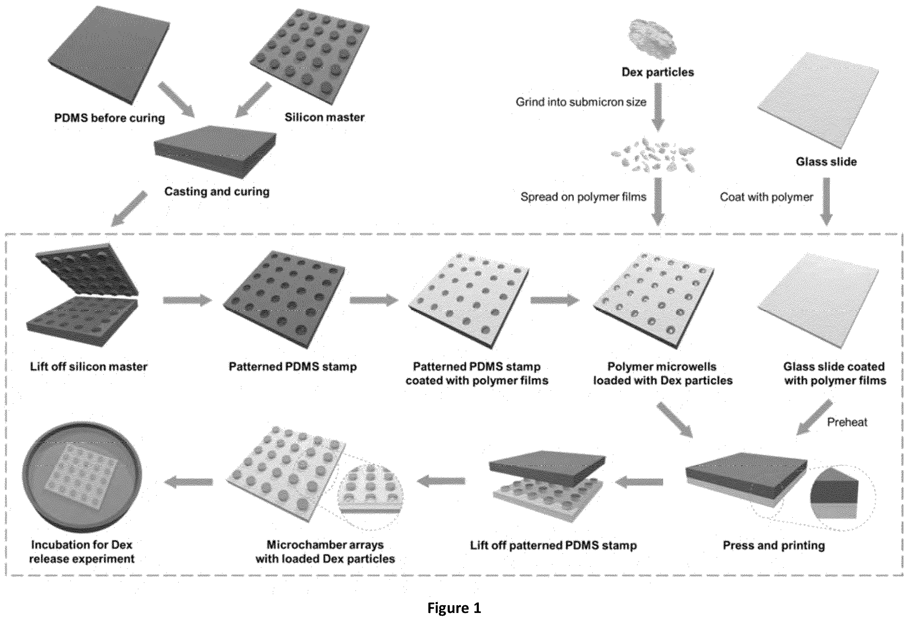

As schematically demonstrated in **Figure 1****,** a PDMS (Polydimethylsiloxane) stamp with the microwell arrays is used for micro-encapsulating crystalline drugs. A layer of polylactic acid (PLA, GoodFellow) film is coated onto the stamp surface by dipping the PDMS stamp into PLA chloroform solution of 1.5% to 3%, which leaves microwell spaces for loading of crystalline drugs. The drug crystals are physically loaded into microwells by spreading drug crystals onto the precoated PDMS surface followed by removing excess crystals, with only a few located near to the ridges between microwells. After that, a glass slide that is also dip-coated with a layer of PLA and dried for 10 seconds, is pressed on the top of the PDMS stamp at the pressure of 0.25 - 1 MPa for 1 minute, thus the crystalline drugs are well-sealed and encapsulated into microchamber array films. The film can be easily detached from both first and second stamp to harvest free-standing microchamber array films.

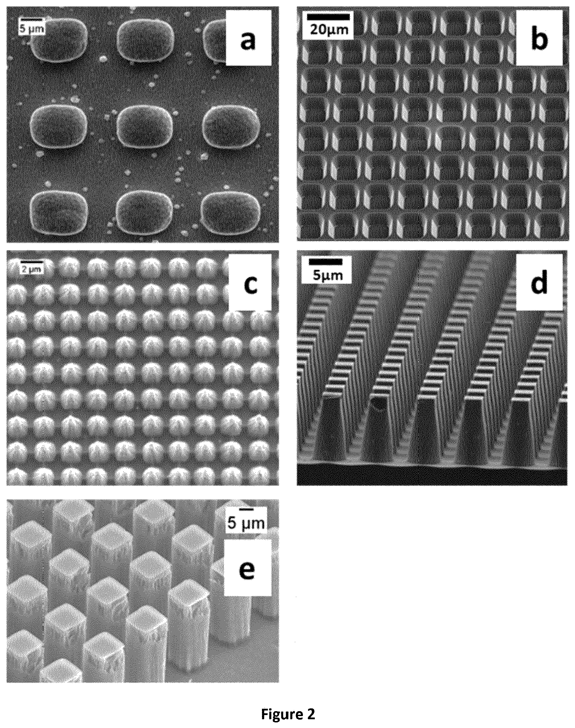

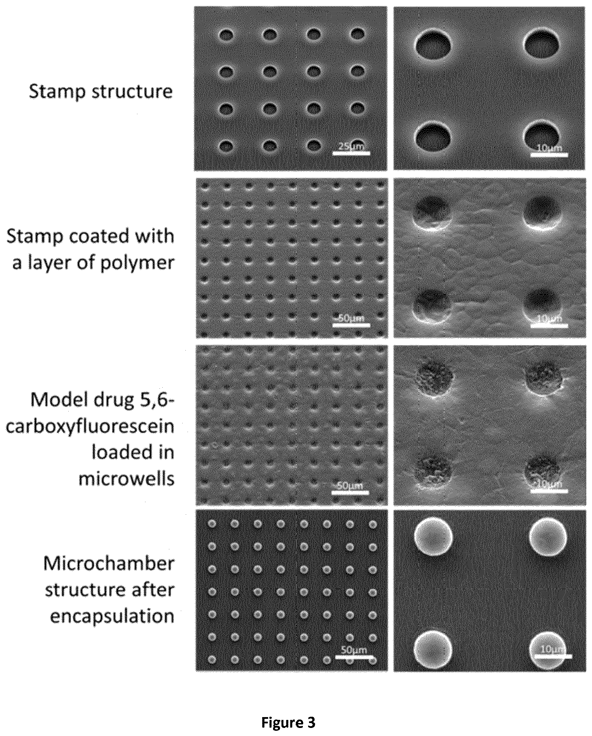

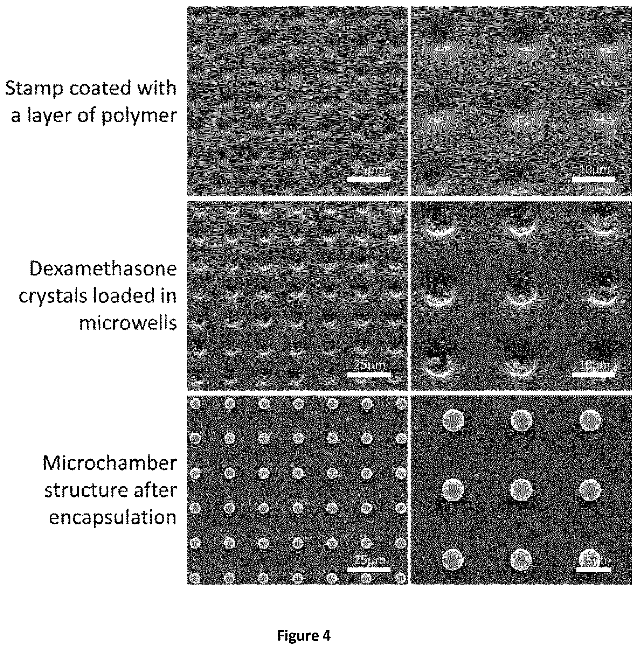

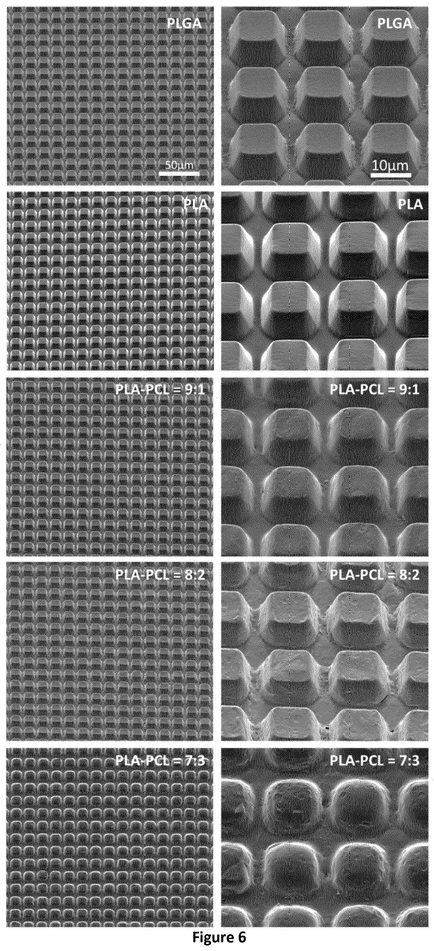

By using stamps with different microarray structures microchamber array films of different microchamber size and shape can be produced. **Figure 2** shows SEM images of micropatterned master stamps with rectangular structures, truncated pyramid structure, stamp with square based pyramids, blunted cones and cuboid. Parameters of each of these stamps are shown in **Table 2.** **Figure 3** demonstrates the microchamber arrays film with cylindrical shape microchambers made with polycaprolactone (PCL), where the loaded crystals were model drug 5,6-carboxyfluorescein. **Figure 4** further shows the cylindrical microchamber arrays films made of PLA with encapsulation of dexamethasone crystals, while the preparation of TP shape microchamber array films is shown in **Figure** 5. Moreover, by using different biodegradable polymer solution, microchamber array films of different polymer contents can be prepared. **Figure 6** shows the SEM (Scanning Electron Microscope) images of PLGA (poly(lactic-co-glycolic acid)), PLA, PLA-PCL = 9:1, PLA-PCL = 8:2 and PLA-PCL = 7:3 TP shaped microchamber array films. Note that with increasing PCL content, the appearance of microchambers becomes more rounded, and the surface morphology tends to be rough. Importantly these arrays can also be prepared with clinical grade polymer as illustrated in **Figure 7** where the TP array was made with PURASORB PDL 05, from Corbion. In this instance dexamethasone crystals are encapsulated and are visible within the microchambers in the optical images (lower panels). In the case of Corbion PLA the solvent used was acetone as opposed to chloroform in previous examples. Nonetheless, the SEM and optical images in **Figure 7** show that the surface morphology of microchamber arrays film made of medical grade polymer is exactly the same as with the natural PLA from GoodFellow.

### Example 2: Controlling the amount of loaded drug

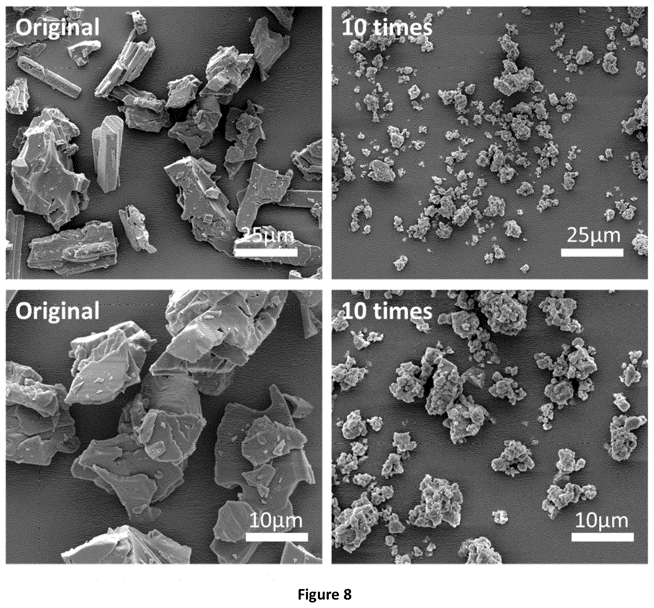

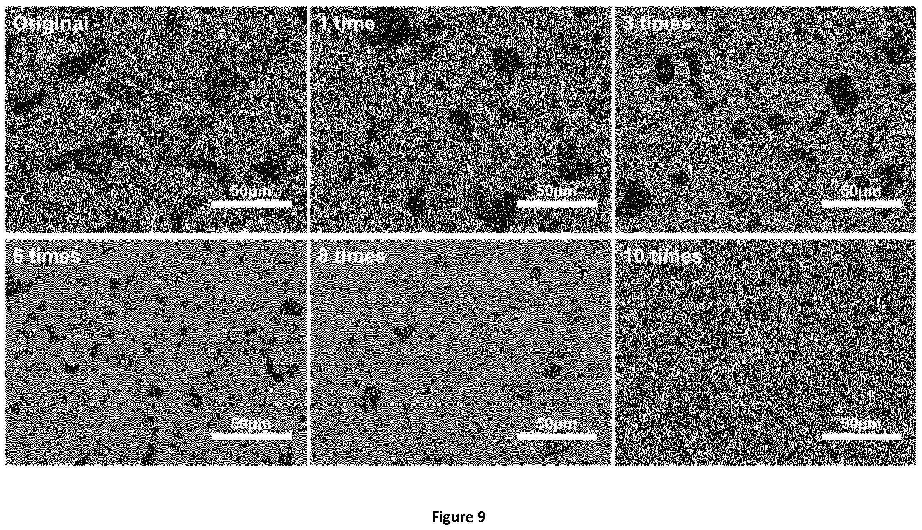

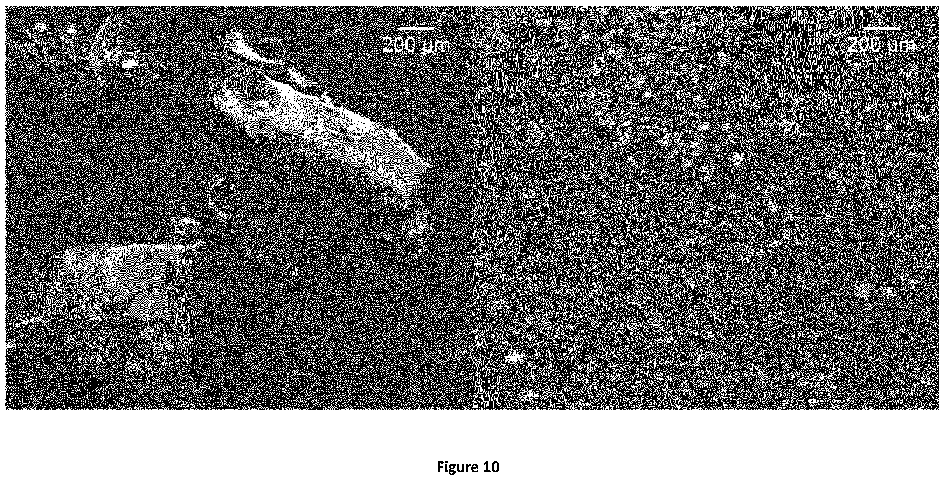

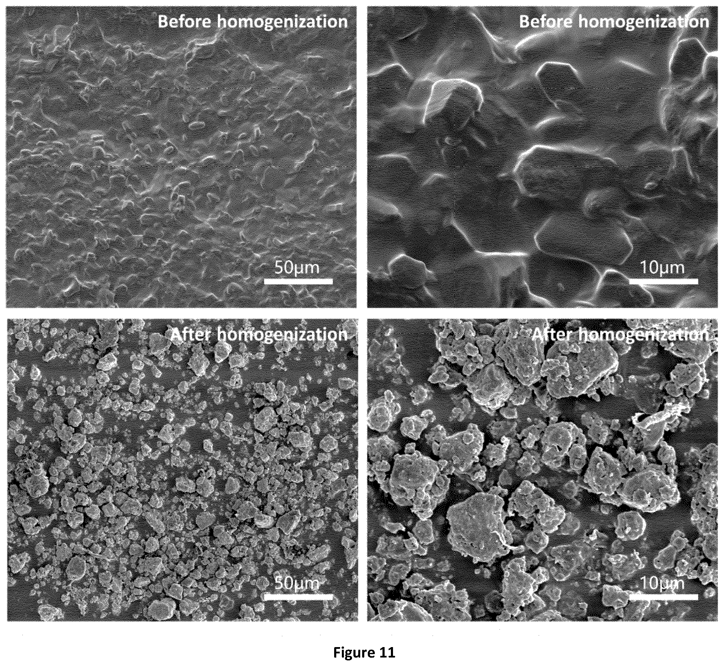

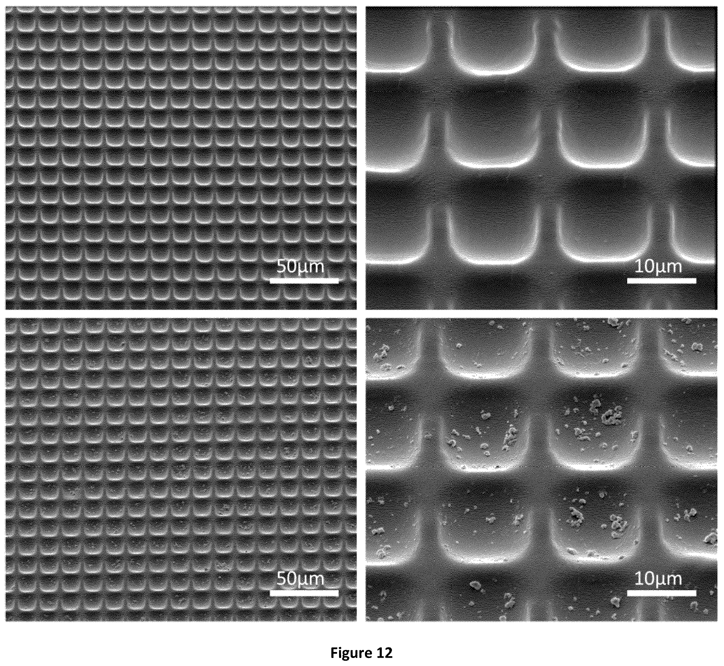

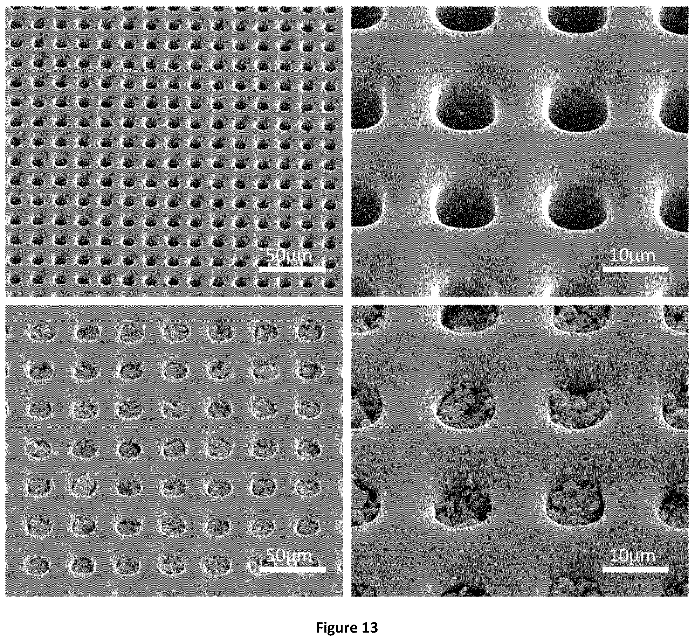

The amount of drug crystals loaded into each microwell on the PDMS stamp is highly dependent on the space left after coating polymer layers. Using lower polymer concentration can lead to thinner film, thus leaving more space for drug crystals. The amount of loaded drug crystals can be further enhanced by decreasing the size of drug crystals using a Precellys homogenizer. SEM and optical images in **Figure 8** and **9** illustrate the size and structure of dexamethasone crystals before and after the homogenization for different times. Each time, the crystals are homogenized at the speed of 5000 for 30 seconds, followed with 2 minutes interval before next time homogenization. In a similar manner two different preparations of catalase from Sigma (C1345 and C30) were homogenised in order to facilitate their encapsulation. The appearance of catalase crystals before and after homogenization are illustrated in **Figure 10** and **Figure 11****.** Loading of homogenised C30 catalase crystals in the TP and cuboidal microchambers is illustrated in **Figures 12** and **13** respectively.

### Example 3: Release kinetics from microchamber array films

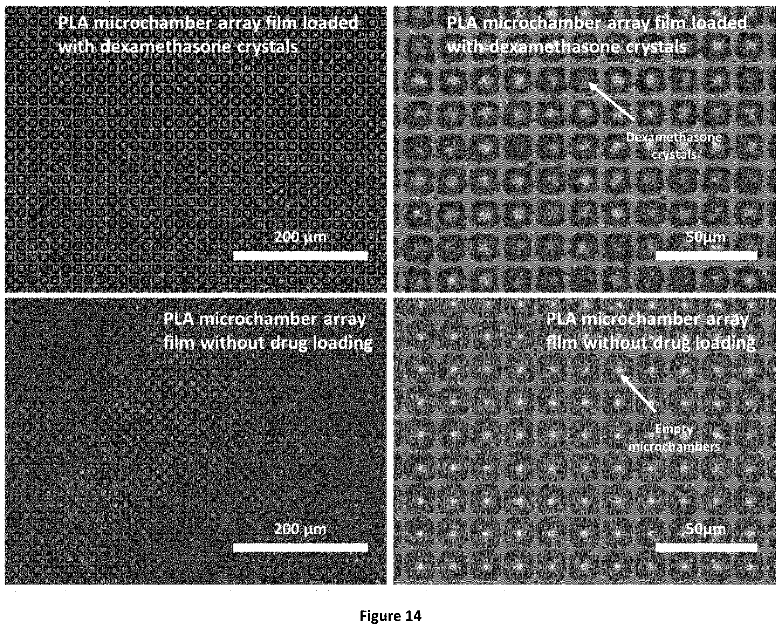

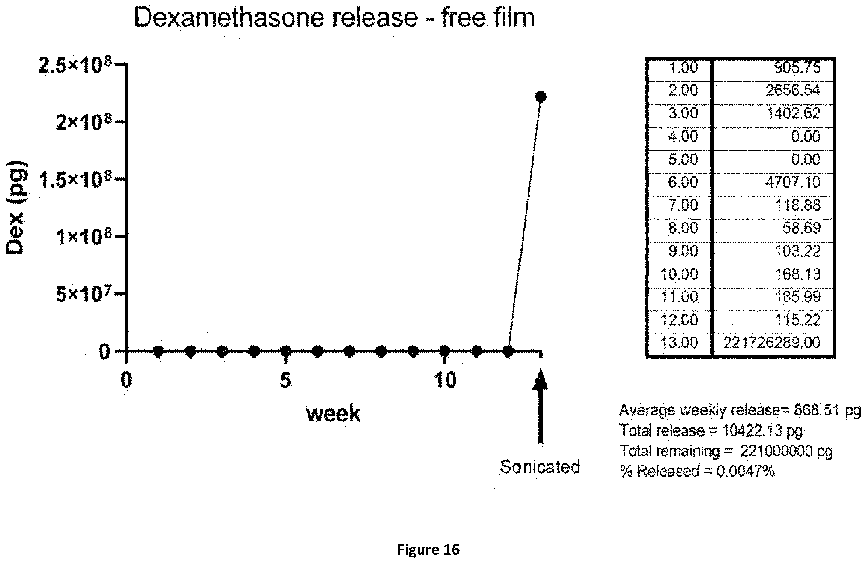

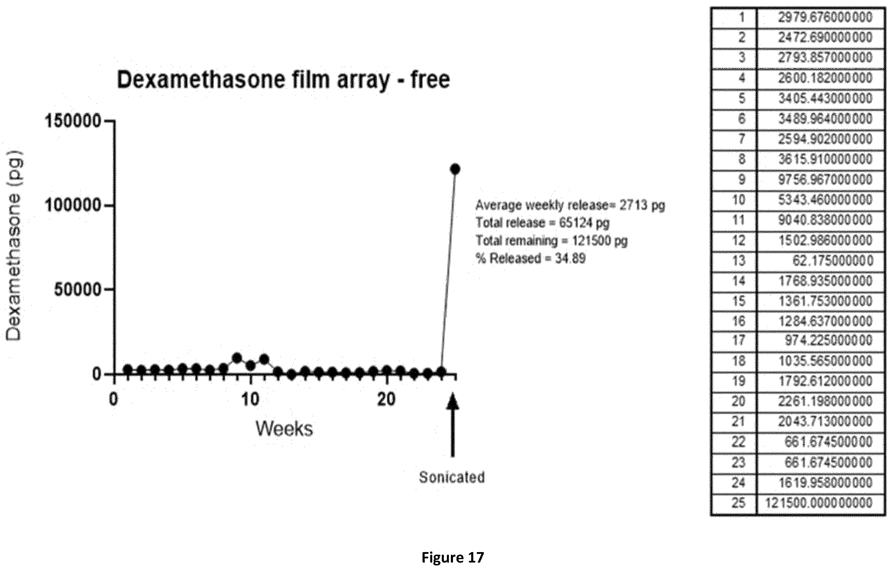

Dexamethasone was effectively loaded into microchambers with the TP shape as shown in **Figure 14****.** When compared to empty chambers in optical images the dexamethasone crystals are clearly visible. We have also loaded dexamethasone labelled with fluorescein into microchambers prepared with the Cylinder 1 stamp. With labelled dexamethasone we were able to monitor its retention following incubation of the PLA (chloroform 2% weight to weight ratio) film in water and then tissue culture media **(****Figure 16** - this is Example 10 below). The observation that labelled dexamethasone was efficiently retained for the duration of the experiment led us to investigate retention of unmodified dexamethasone in the same microchamber array in two subsequent studies. In one **(****Figure 16** - this is Example 11 below) the array was incubated for 12 weeks and in the second **(****Figure 17** - this is Example 12 below) the film was incubated for 24 weeks. The efficient retention of glucocorticoid in these longer studies clearly illustrated the uniqueness of the these microchamber arrays for retention of crystals of insoluble drug.

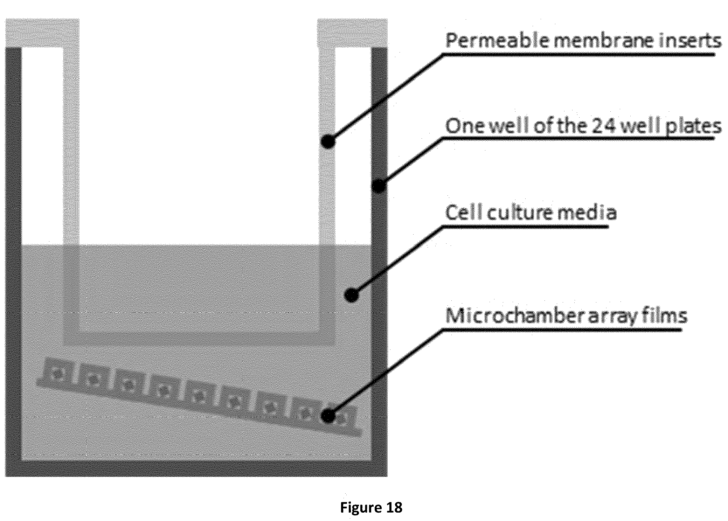

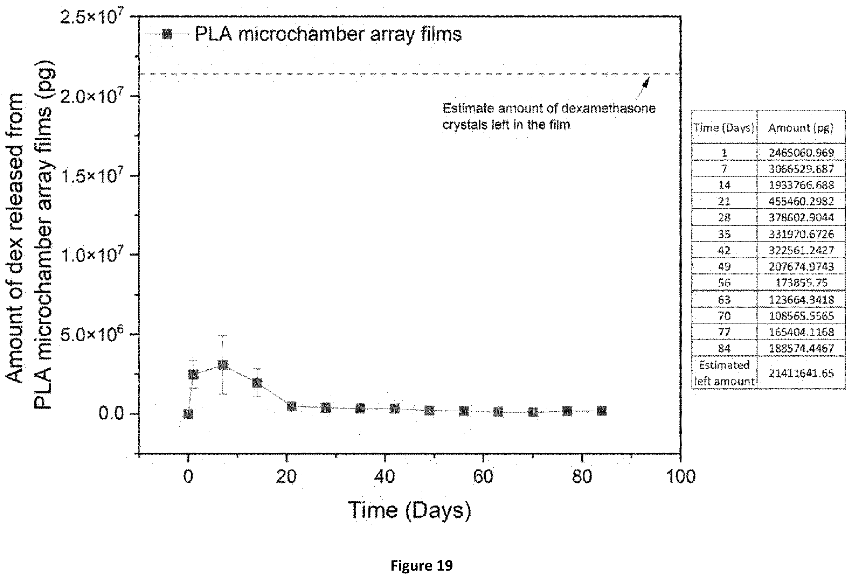

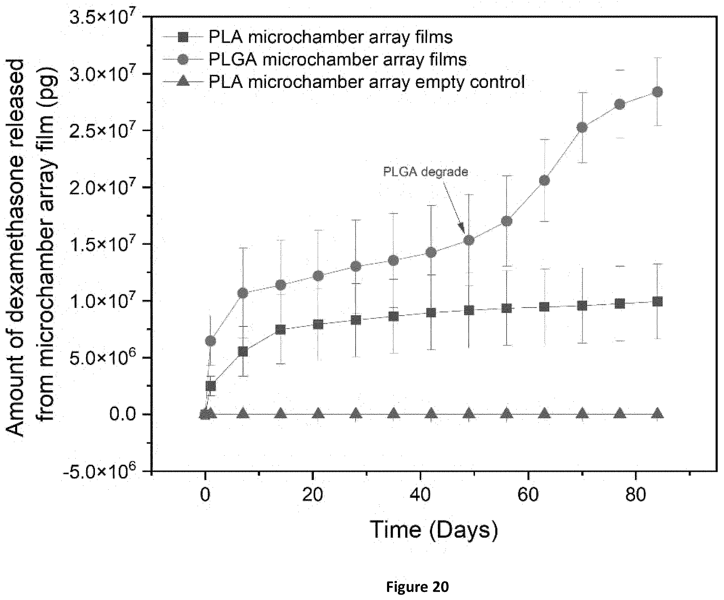

We have extended these observations to the TP microchamber array film. **Figure 18** shows a schematic of the release experiment similar to earlier release experiments. As microchamber array films float in releasing media, it is important to keep the film fully submerged. Here we use inserts with permeable membranes to keep the film in the releasing media. Generally, the microchamber array films were placed into the wells of 24-well plates. Afterwards, inserts were put into each well, followed with addition of 1.5 ml of releasing media. The 24-well plates were incubated in a humidified tissue culture incubator with the temperature set at 37°C and with 5% CO₂. During the release study, the microchamber array film was incubated in 1.5 ml of media which was replaced on day 1, day 7, and then weekly media replacement. Dexamethasone release kinetics from the TP microchambers is shown in **Figure 19** and **Figure 20****.** **Figure 19** demonstrates the amount of dexamethasone released from the TP shape PLA microchamber array films during the 12 week releasing studies, where an estimated 31.7% of dexamethasone was released during the study and the dotted line indicates the amount that remained after 12 weeks. Alternatively, when the microchamber array films were prepared using PLGA cumulative release of dexamethasone is illustrated in **Figure 20** and compared to PLA and empty PLA microchambers. Release of dexamethasone from the PLGA microchamber array films accelerated after week 7, which is induced by the degradation of PLGA while the PLA film displayed a low release rate. As expected the control group of PLA microchamber array film without dexamethasone showed no dexamethasone release. The observations from this experiment are important because they indicate the potential of controlling of the kinetics of dexamethasone release through manipulation of the microchamber polymer composition.

### Example 4: Pressure method for generation of microcapsules

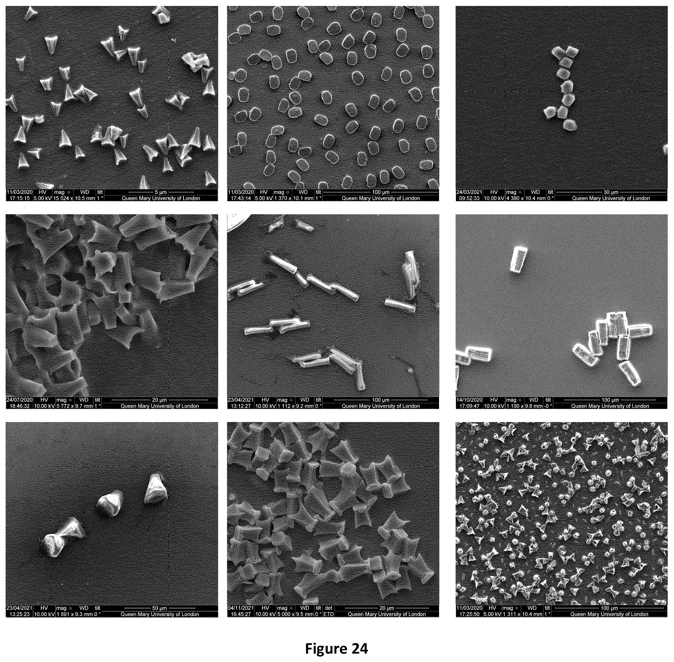

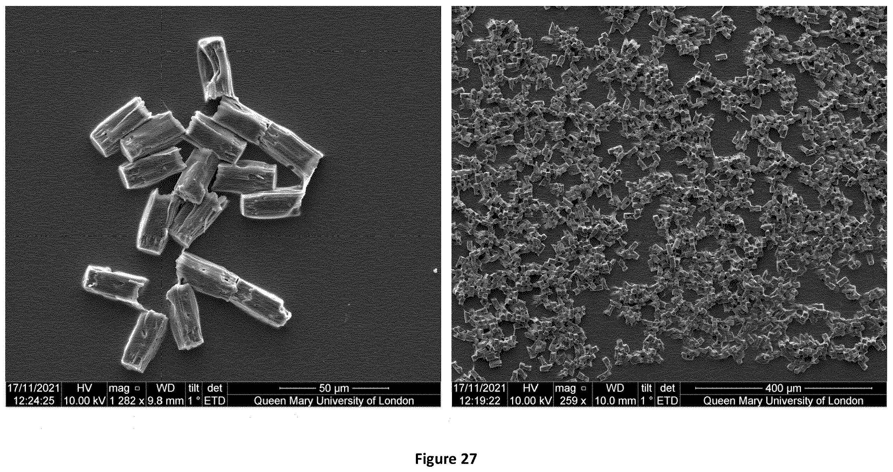

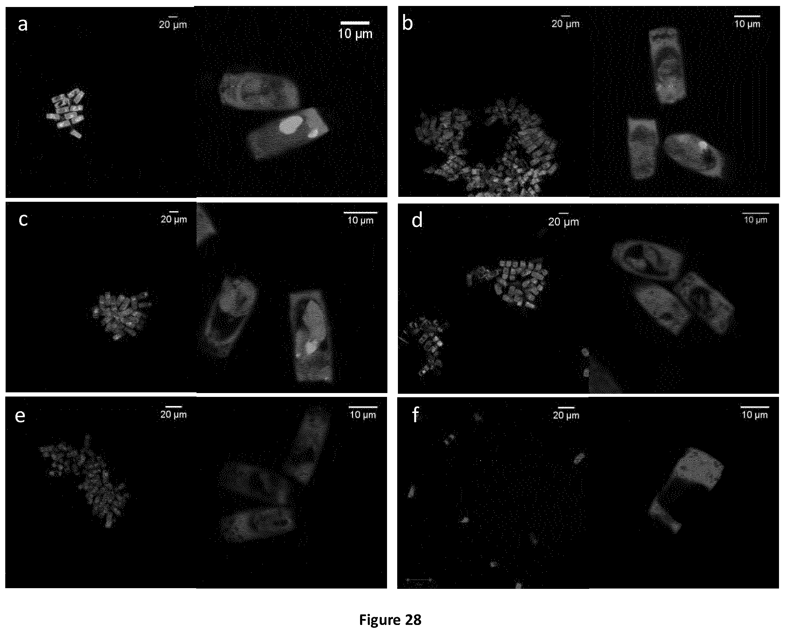

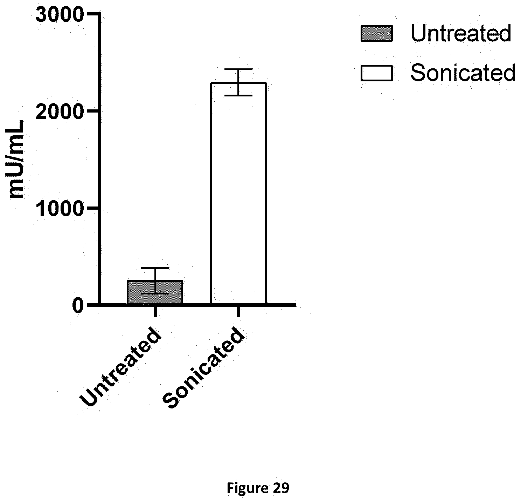

Several stamps have been used to generate microcapsules by the pressure method. Indeed **Figure 23** shows images of rectangular, pyramid and blunted cone capsules prepared with PLA solution by pressure method. The versatility of this capsule fabrication method is illustrated by the range of different capsule shapes presented in the **Figure 24****.** The range of the size of capsules is from 1.5 µm up to 40 µm and the parameters of the stamps used to prepare these capsules are shown in **Table 2.** The different compartments of the microcapsule are illustrated in **Figure 25** where a single rectangular microcapsule is constructed with labelled PLA shell of the capsule (a1 in red) and is loaded with a cargo of 5,6-carboxyfluoresceine (a2 in green), combined in the merged image (a3) and observed under bright field (a4). FIB-SEM in combination with BSE detector allows even better visualization of the interior part of the produced microcapsules. **Figure 26a** shows a capsule filled with several FeCl₂ crystals, where a free space is evident inside the capsule (red arrows), while **Figure 26b** illustrates an even distribution of Fe₃O₄ nanoparticles inside the capsule and a very high-volume fraction of the encapsulated substance. The smaller Fe₃O₄ crystals allow almost complete filling of the internal volume. In **Figure 27** shows images of PLGA microcapsules prepared with pressure method with cuboid shape and size of 11×11×22 µm. Importantly we have been able load these PLGA microcapsules with homogenised catalase crystals **Figure 28** which are shown in CLSM images (PLGA capsules (red) with encapsulated catalase (green)). These microcapsules were imaged on day 0, day 1, day 3, day 6, day 14 and confirming the retention of catalse crystals. On day 14 the microcapsules were ultrasonicated with a Fisherbrand^{™} Model 120 on ice which triggered catalase release. This study proves that capsules can be used for encapsulation and storage of sensitive cargoes such as enzymes. Furthermore, **Figure 29** shows that catalase activity from untreated and sonicated PLGA microcapsules (150 000 capsules per ml) in 1 ml was measured by Amplex ^{™} Red assay. Before treatment of capsules catalase showed low activity, but sonication released the catalase cargo resulting in a ten times increase in activity after the treatment. This data shows that the pressure method can be used to encapsulate enzyme (protein) and upon release it remains active.

### Example 5: Preparation of biodegradable crystalline drug loaded microcapsules with heat-press

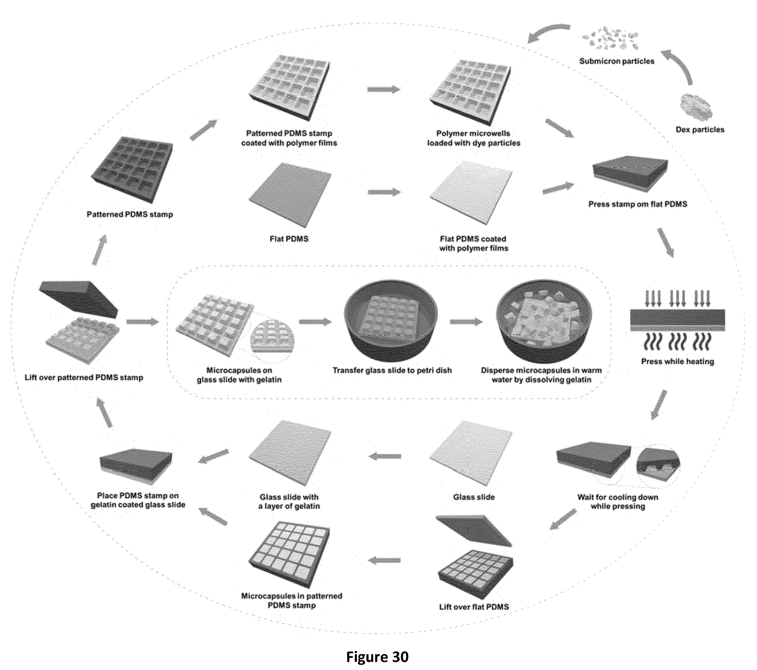

As schematically illustrated in **Figure 30****,** a PDMS stamp with the microwell array is used for micro-encapsulating crystalline drugs. A layer of polylactic acid (PLA) film is coated onto the stamp surface by dipping the PDMS stamp into PLA chloroform solution of 1.5% to 3%, while still leaving microwell spaces for loading crystalline drugs. The drug crystals are physically loaded into microwells by spreading drug crystals onto the precoated PDMS surface followed by removing the excess crystals, with only few located near to the ridges between microwells. After that, a flat PDMS substrate that dip-coated with a layer of PLA and dried for 60 seconds, is covered on the top of the PDMS stamp then, both the PDMS stamp and the covered flat PDMS substrate are pressed at the pressure of 0.25 - 1 MPa while heated at the 140-200°C for 1 - 3 minutes. Note that the temperature and time vary with the polymer employed as long as the polymer undergoes phase transition or starts to melt. Afterwards, the PDMS stamp is cooled down to room temperature at ambient conditions while still covered by the flat PDMS substrate. Only when the PDMS stamp is fully cooled down can the flat PDMS substrate be gently detached from the PDMS stamp, leaving individual microcapsules embedded into each microwells of the PDMS stamp. In order to harvest the microcapsules suspension, a 0.2 to 1 ml of 10% weight to weight gelatin water solution is spread on the glass slide, whereas the microcapsule embedded PDMS stamp is put onto the gelatin carefully avoid trapping any air bubbles. Then, both the PDMS stamp and the gelatin coated glass slide are moved to -20°C freezer for 10 to 30 minutes in order to solidify the gelatin solution. In the next step, the PDMS stamp and the glass slide are taken out from the freezer and separated immediately so that all the embedded microcapsules are transferred onto the glass slide by the gelatin. Finally, the PLA microcapsule water suspension is prepared by dissolving the gelatin into 37°C warm water followed by 3 to 6 times washes to remove the gelatin. A washing step is performed by centrifuging the microcapsule water suspension at 5000 rcf for 1 minute, replacing the supernatant, and resuspending the microcapsules.

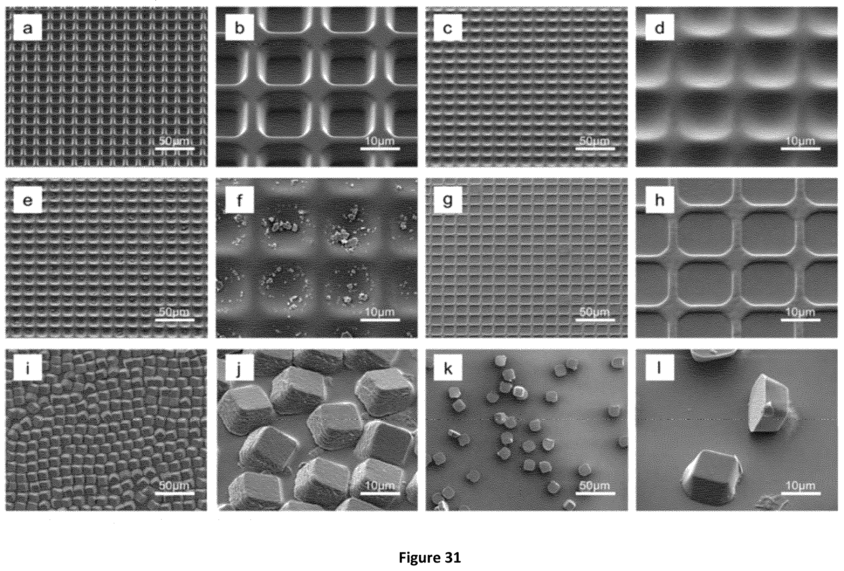

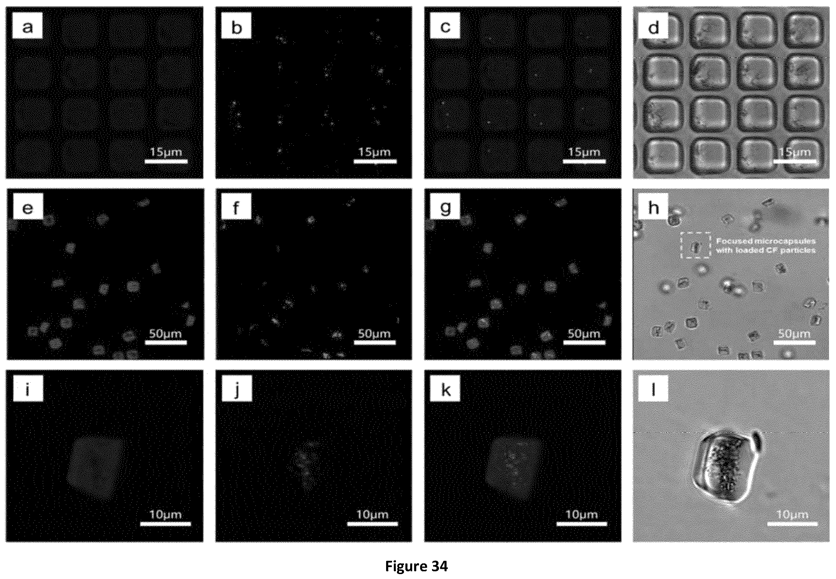

SEM images of the key steps of the microcapsules preparation have been organized in **Figure 31****.** Moreover, **Figure 34** shows the confocal laser scanning microscope (CLSM) images of the microcapsules embedded in microwells of the PDMS stamp as well as the microcapsules after dispersion in water

The processing temperature can also significantly influence the preparation of PLA microcapsules. As shown in **Figure 32****,** when the heat-press temperature was 120°C (much lower than the phase transition or melting point of the polymer), the microcapsules are not formed and instead, microchamber array films are fabricated. When temperature goes above 140°C, microcapsules can be fabricated as usual.

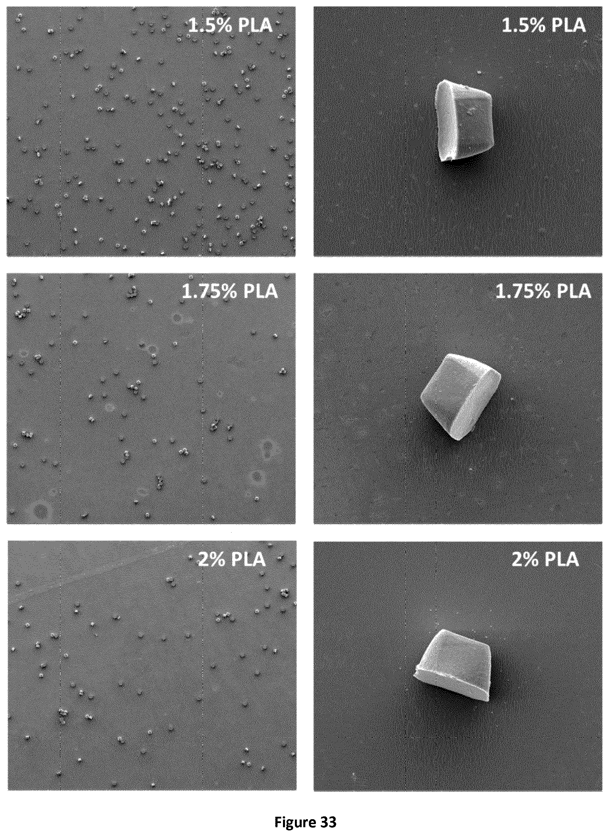

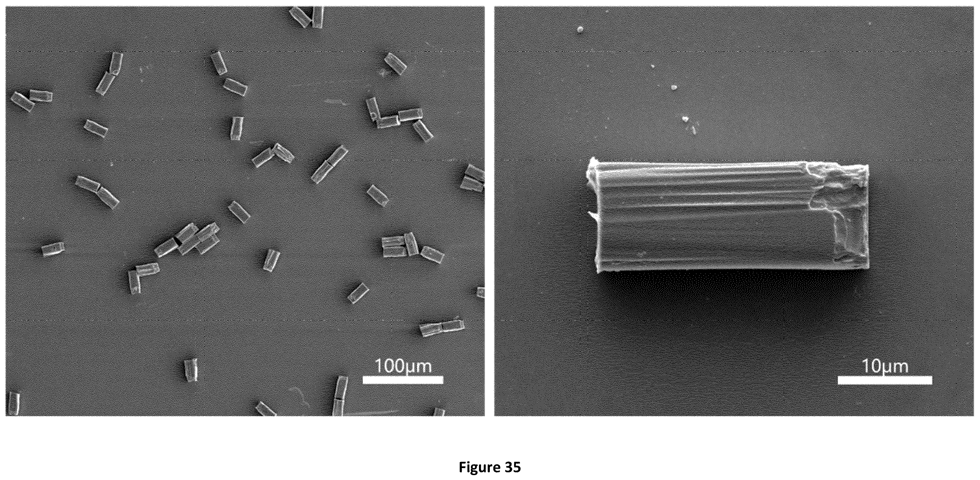

By using different concentrations of biodegradable polymer solution, microcapsules of different polymer contents can be prepared. **Figure 33** shows the SEM images of microcapsules made by chloroform solutions of 1.5% PLA, 1.75% PLA and 2% PLA. By using different templates, the size and shape of microcapsules can be manipulated. **Figure 31 k, l****)** and **Figure 35** show the SEM images of TP and cuboid shape PLA microcapsules.

### Example 6: Characterization of heat press microcapsules

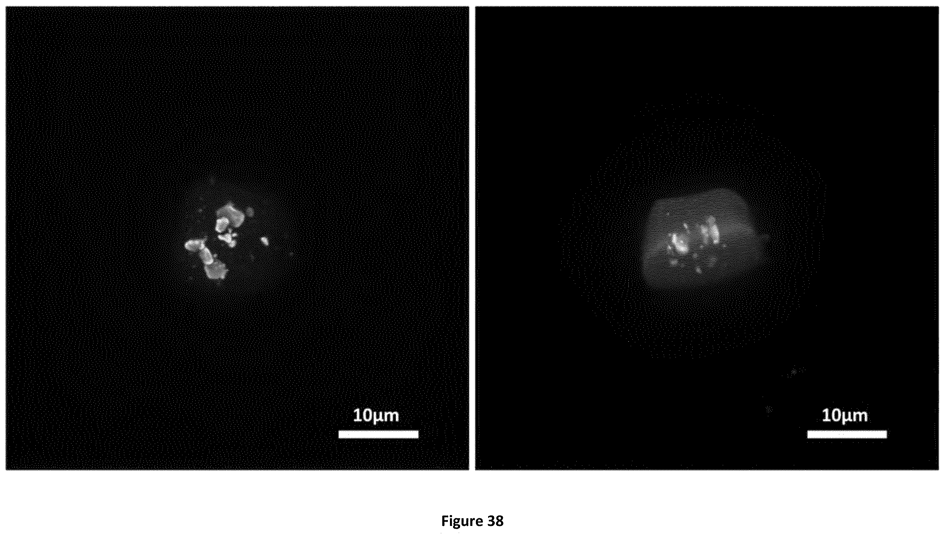

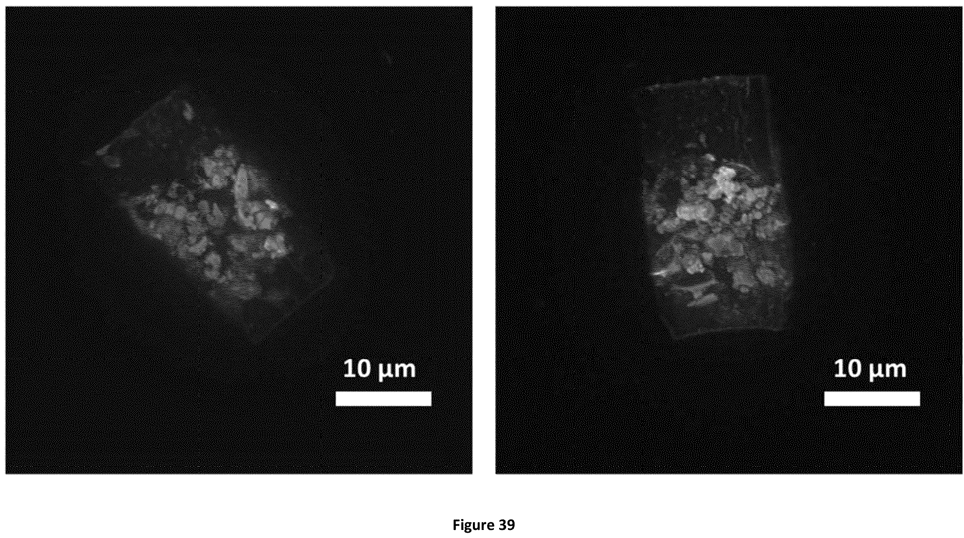

**Figure 33** shows the SEM images of microcapsules made by chloroform solutions of 1.5% PLA, 1.75% PLA and 2% PLA. The morphology of microcapsules made of PLA of different concentration do not have distinct difference, as all the surface of microcapsules are complete with same size and structures. **Figure 31 k, l** and **Figure 34** show the specification of the microcapsules of truncated pyramid and cuboid shape microcapsules, respectively. The polymer of PLA was labelled with Nile red showing red fluorescent colour, while the dexamethasone was labelled with fluorescein and can be observed as green colour under the CLSM. It is clearly observed that the dexamethasone crystals are encapsulated into the microcapsules in solid crystals state, and fully wrapped by the shell polymer. This can also be confirmed with transmitted optical images in **Figure 38** and **Figure 39****,** where drug crystals are apparently located inside the microcapsules. It is noteworthy to address that all the samples above except SEM are characterized in water suspensions, which also an indication of the well-encapsulation as the crystals would diffused into water if not well-sealed.

### Example 6: Encapsulation of protein crystals in heat-press PLA microcapsules

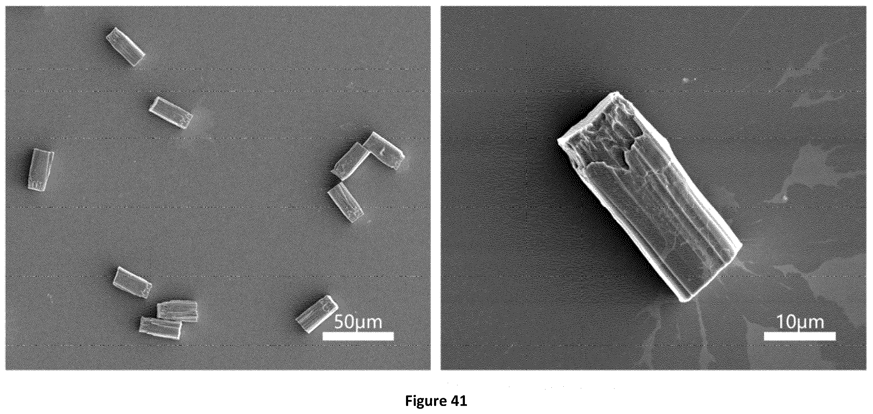

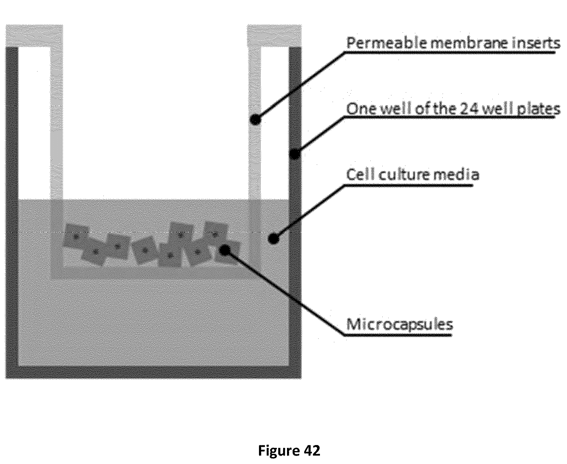

The optical image and SEM images of PLA microcapsules loaded with catalase crystals are shown in **Figure 42** and **Figure 41****,** respectively. Generally, the catalase crystals loaded PLA microcapsules has exactly same morphology as the dexamethasone crystal loaded ones. Moreover, the optical images also support that the crystal catalase were well encapsulated inside microcapsules.

### Example 8: The release kinetics of dexamethasone from heat-press microcapsules

As shown in **Figure 42****,** 100,000 dexamethasone PLA microcapsules were put into an insert with permeable membrane, where the inserts fit into 24 wells plates. 1 ml of releasing media was added into wells so that the microcapsules were immersed into the media while not dispersed into the wells. The solution was periodically replaced with new releasing media at specific time points, the amount of dexamethasone in the release samples was measured using the reporter cell assay.

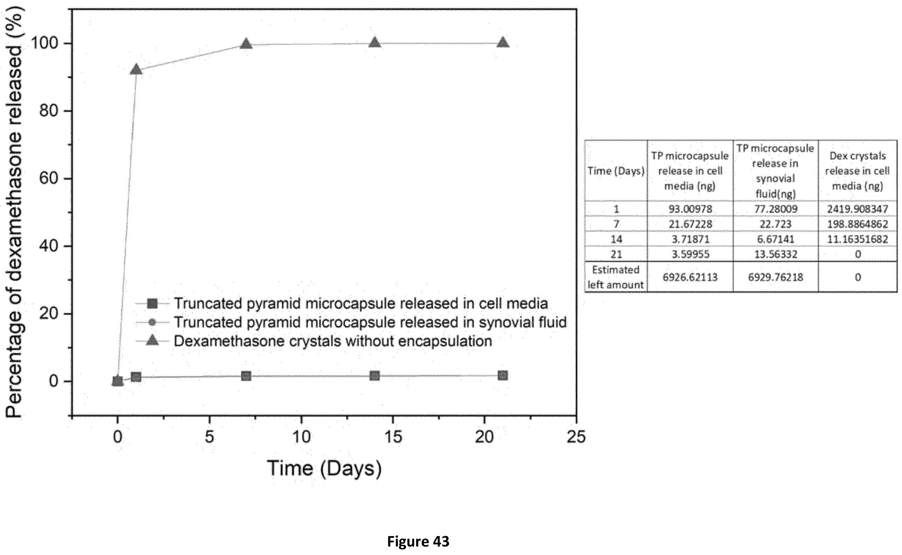

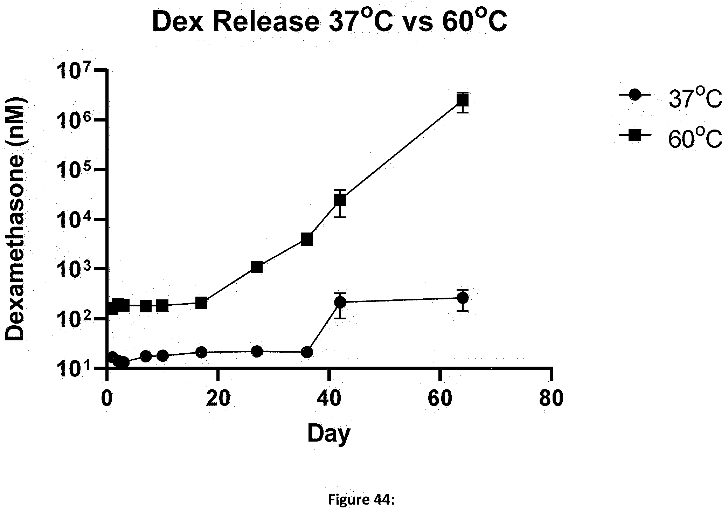

**Figure 43** illustrates the percentage of dexamethasone released in 3 weeks from dexamethasone crystals (2.6 µg) without encapsulation and from 100,000 TP microcapsules (estimated total dexamethasone 7.05 µg) in cell culture media (n=3) or human synovial fluid (n=1). In the group, dexamethasone crystals display almost 95% released within the first 24 hours, and the dexamethasone is fully released after day 7. In contrast, only about 2% of dexamethasone was released after 21 days for TP microcapsules. In general, the release profiles of TP microcapsules are similar in both cell culture media and synovial fluid. Cumulative release of dexamethasone into water at 37°C and 60°C was also monitored. 10,000 TP shape microcapsules in 500 µl of water were incubated at 37 °C or 60°C. 50 µl of water was collected at the indicated timepoints and volume replaced with distilled water. Dexamethasone content of the collected water samples was determined in the responsive cell assay and the data confirms low level release at 37°C whilst release can be expedited by incubation at 60 °C (**Figure 44****).**

### Example 9: Shapes and dimensions of the microchambers

Table 2 below provides examples of the shape and dimensions of different microchambers prepared according to the present invention, and the correlation with the provided Figures.

**Table 2**

| **Number** | **Microchamber shape** | **Microchamber dimensions (µm)** | **Template periodicity (µm)** | **Microcapsules prepared** | **Relevant Figures** |
|---|---|---|---|---|---|
| 1 | Cuboid (C) | 11×11×30 | 20 | Yes | Stamp 2e |
| | | | | | Microchambers 13 |
| | | | | | Microcapsules 27, 28, 29, 35, 37, 39, 40, 41 |
| 2 | Truncated pyramid (TP) | 10 (short length) x 14 (long length) x 8 (height) | 15 | Yes | Stamp 2b |
| | | | | | Microchambers 5, 6, 7, 12, 14, 19, 20, 21 |
| | | | | | Microcapsules 31, 32, 33, 34, 36, 38, 43 and 44 |
| 3 | Cylinder 1 | 4x10 Ø | 30 | No | Microchambers 3, 4, 15,16, 17 |
| 4 | Rectangular | 14×9×4 | 20 | Yes | Stamp 2a |
| | | | | | Microcapsules 23a+d, 24, 25 |
| 5 | Square based pyramids | 1.8×2 | 2.5 | Yes | Stamp 2c |
| | | | | | Microcapsules 23b+e |
| 6 | Blunted cones | 9x5x3 | 7 | Yes | Microcapsules 23 c+f, 24 |
| 7 | Cylinder 2 | 40x10 Ø | 25 | Yes | Stamp 2d |
| | | | | | Microcapsules 24 |
| 8 | Small cuboids | 4x4 | 10 | Yes | Microcapsules 24 |
| 9 | Cuboids with hat | 5x3x3 | 10 | Yes | Microcapsules 24 |
| 10 | Big pyramids | 15x15Ø | 20 | Yes | Microcapsules 26 |
| 11 | Small pyramids | 10x8 Ø | 12 | Yes | Microcapsules 24 |

### Example 10 - Release of fluorescent dexamethasone

PLA microchamber films were prepared containing dexamethasone FITC as cargo as shown in Figure 1. The dry film was examined under fluorescence microscopy and image 1 captured. The film was incubated in 50 ml of sterile water at room temperature for 5 weeks. After 4 weeks the film was removed from the water and a florescence microscope image captured (Image 2). The film was returned to the water and incubation continued for another week after which another fluorescence microscopy image of the film was taken (Image 3) and the water was analysed for measurement of dexamethasone content. The film was then transferred to 50 ml of sterile complete cell culture media (Dulbecco's Modified Eagle medium (DMEM) supplemented with 10% fetal calf serum, 1% penicillin-streptomycin and 1% L-glutamine) and was again incubated at room temperature for a further 4 weeks. The media was changed every week and the film was again imaged after weeks 6 and 7 (Images 4 and 5 respectively). The images clearly show that fluorescent dexamethasone is still present in the microchambers at the end of the 9 week incubation.

The amount of dexamethasone released into the water (50 ml) after 5 weeks of incubation and into the media each week (wks 6, 7, 8 and 9) was quantitated with a responsive cell line. At the end of the study (wk 9) the film was sonicated in 2 ml of water and the amount of dexamethasone released was again quantified using the responsive cell line.

A glucocorticoid responsive 293T cell line engineered by lentiviral infection to contain a synthetic glucocorticoid responsive promoter driving expression of the firefly luciferase gene was used for the determination of glucocorticoid levels in samples (Read et *al*., 2020). Cells were plated in 96 well plates and the next day were incubated with dexamethasone standards and samples (undiluted or diluted with complete media). Luciferase activity in cell lysates treated with samples and standards were used to determine dexamethasone levels in samples. The assay is accurate down to dexamethasone concentrations of 1 nM (392.46 pg/ml). Using this assay 742.6 ng of dexamethasone was shown to be released during the 5 week incubation in water, dexamethasone was undetectable in the media collected from weekly incubations (measurement attempted for wks 6, 7 and 9) and upon sonication at the end of wk 9, 75 µg of dexamethasone was released. These results clearly indicate that the majority of the dexamethasone remained trapped in the microchamber film for the duration of the experiment. We postulate that the dexamethasone detected in the water incubation may be the result of steroid that was superficially associated on the film. In subsequent experiments films were washed with water before dexamethasone release was examined.

### Example 11 - Dexamethasone release at 37°C

Dexamethasone (non-fluorescent) was incorporated into the second microchamber film, which was incubated in 50 ml of water over the weekend to remove superficial dexamethasone. The film was then transferred to 1 ml of sterile water in the well of 24 well plate, a transwell insert was placed in the well in order to ensure that the film remained submerged for the duration of the experiment and the incubation was performed in a tissue culture incubator (37°C/5% CO₂/humidified atmosphere). The film was incubated in water for 5 weeks and at the end of each week 300 µl of the water was removed and replaced with the same volume of sterile water. After 5 weeks, incubation continued in 1 ml of complete cell culture media and now the 1 ml volume was changed every week. Incubation continued until 12 weeks. At this time the film was sonicated in 3 ml of sterile water to release the remaining dexamethasone. The wash sample, the water and media release samples and the sonication samples were then assessed for dexamethasone content using the glucocorticoid responsive 293T cell line.

Results showed that 8338.5 ng of dexamethasone was released from the film during the weekend wash; during the course of the release experiment the average weekly release of dexamethasone was 868.51 pg and upon sonication a further 221 µg of dexamethasone was released from the film (Figure 2). The experiment confirmed that a small amount of dexamethasone is superficially associated with the microchamber array when it is manufactured. Dexamethasone is slowly released from the film during incubation and after 12 weeks the vast majority of the dexamethasone still remains within the microchambers.

### Example 12 - Dexamethasone release over 24 weeks

A third dexamethasone film array was washed in 50 ml of sterile water for 3 months before assessment of dexamethasone released in the release experiment. The film was then transferred to 1 ml of complete media in a well of a 24 well plate, a transwell insert was again placed in the well in order to ensure that the film remained submerged for the duration of the experiment. The 1 ml of complete cell culture media was changed every week for fresh media and the collected sample stored for dexamethasone analysis. Incubation continued until 24 weeks. At this time the film was sonicated in 3 ml of sterile water to release the remaining dexamethasone. The media release samples and the sonication samples were then assessed for dexamethasone content using the glucocorticoid responsive 293T cell line. Results showed that the average weekly release of dexamethasone from the film was 2713 pg and that 121500 pg was released upon sonication at the end of the release experiment (Figure 3).

Key observations
1. The results obtained with dexamethasone microchamber film arrays have consistently shown a slow rate of dexamethasone release even in the release experiment up to 24 weeks.
2. In all experiments the majority of the dexamethasone remained in the film at the end of the experiment.
3. A certain amount of dexamethasone is loosely associated with the microchamber film when it is manufactured and this can be washed away relatively easily.
4. The dexamethasone microchamber film arrays are very stable, they can be stored for several months, even in water, and show similar release kinetics when utilised in release experiments.

### REFERENCES

Read JE, Luo D, Chowdhury TT, Flower RJ, Poston RN, Sukhorukov GB, Gould DJ. Magnetically Responsive Layer-By-Layer Microcapsules Can Be Retained in Cells and Under Flow Conditions to Promote Local Drug Release Without Triggering ROS Production. Nanoscale. 2020 Apr 14;12(14):7735-7748. doi: 10.1039/c9nr10329e.

## Claims

1. A method for micro-encapsulating a crystalline drug, comprising:
a. providing a first stamp comprising a plurality of microwells, wherein the microwells of the first stamp are coated with a polymer composition comprising polylactic acid (PLA), polylactic-co-glycolic acid copolymer (PLGA), or a blend thereof;
b. loading crystalline drug into the coated microwells;
c. providing a second stamp, wherein the second stamp is planar and is coated on at least one side with the polymer composition comprising polylactic acid (PLA), polylactic-co-glycolic acid copolymer (PLGA), or a blend thereof; and
d. pressing the coated sides of the first and second stamps together to encapsulate the crystalline drug in the wells with the polymer composition to form a film of microchambers.

2. The method of claim 1, wherein the first and/or second stamps are coated with a polymer composition layer having a thickness of from about 0.05 to about 2 microns.

3. The method of claim 1 or claim 2, comprising providing the first and second stamps, and coating the stamps with a solution comprising the polymer composition and evaporating the solvent of the polymer solution, optionally wherein the polymer solution has a concentration of from about 0.1% to about 25%, or from about 0.1% to about 5%, or from about 0.5 to about 2%, or about 1%.

4. The method of any one of claims 1 to 3, further comprising removing the film of microchambers from the first stamp.

5. The method of any one of claims 1 to 3, wherein
(a) the method comprises pressing the coated sides of the first and second stamps together at a pressure of up to about 0.25 MPa (or up to about 0.1 Mpa) and the method does not comprise a step of heating the polymer composition,
optionally further comprising separating the first and second stamps and of removing excess polymer composition from the first stamp, wherein the excess polymer is removed by mechanical action, for example scraping, and wherein the step of removing excess polymer composition separates the microchambers into separate microcapsules in the wells of the first stamp, and further comprising removing the microcapsules from the first stamp; or
(b) wherein the step of pressing the stamps together comprises heating the polymer composition to a temperature that is greater than a temperature that is 20 °C below the melting point of the polymer composition, and wherein the method comprises pressing the coated sides of the first and second stamps together at a pressure of at least about 0.1 MPa, wherein the step of pressing the stamps together while heating separates the microchambers into separate microcapsules in the wells of the first stamp, and further comprising removing the microcapsules from the first stamp;
further optionally
(i) wherein the step of removing the microcapsules from the first stamp comprises:
a. providing a planar substrate coated with a soluble adhesive;
b. pressing the planar substrate onto the first stamp to adhere the microcapsules to the slide;
c. removing the microcapsules from the microwells of the first stamp by separating the planar substrate and the first stamp; and
d. dissolving the adhesive to separate the microcapsules from the planar substrate; and/or
(ii) wherein the method further comprises formulating the microcapsules into an injectable pharmaceutical composition with one or more pharmaceutically acceptable excipients.

6. The method of any one of claims 1 to 5, wherein the microcapsules or microchambers comprise a core and a shell, wherein the core comprises a crystalline drug and the shell comprises polylactic acid (PLA) or PLGA, and wherein the shell completely encapsulates the core.

7. The method of claim 6, wherein the microcapsules or microchambers core consists of the crystalline drug and optionally air.

8. The method of claim 6 or 7, wherein the crystalline drug has a solubility of less than 1mg/ml in water at 25 °C and has a melting point above about 200°C.

9. The method of any one of claims 6 to 8, wherein the drug is a corticosteroid, optionally wherein the corticosteroid is selected from the group consisting of dexamethasone, prednisolone, betamethasone, prednisone, methylprednisolone, budesonide, hydrocortisone, triamcinolone and fludrocortisone, or wherein the drug is a protein, optionally wherein the protein is a growth factor, an enzyme, a cytokine, a chemokine or a biological therapeutic;
optionally wherein the drug is dexamethasone.

10. The method of any one of claims 6 to 9, wherein:
(a) the shell has a melting point of from about 50 °C to about 200 °C; and/or
(b) the shell comprises PLA homopolymer, PLGA, or a PLA/PLGA blend, and optionally polycaprolactone (PCL), optionally wherein:
(i) the shell consists of PLA homopolymer; or
(ii) the shell consists of PLA and PCL and the shell has more PLA than PCL by weight.

11. A sustained-release composition comprising a plurality of microcapsules obtained or obtainable by the methods of any one of claims 5(a) to 6, or comprising a film of microchambers obtained or obtainable by to the methods of any one of claims 1 to 5(a).

12. The sustained-release composition of claim 11, wherein
(a) the composition further comprises one or more pharmaceutically acceptable excipients or diluents, optionally wherein the sustained-release composition comprises PBS; and/or
(b) wherein the composition is injectable.

13. A sustained-release composition according to claim 11 or claim 12 for use in the treatment of a disease or disorder,
optionally wherein the disease or disorder is an inflammatory disease or disorder, for example arthritis.

14. A drug-eluting implantable or injectable medical device comprising or consisting of a film of microchambers defined according to claim 11.

## Patentansprüche

1. Verfahren zur Mikroverkapselung eines kristallinen Arzneistoffs, umfassend:
a. Bereitstellen eines ersten Stempels, der eine Mehrzahl an Mikrokavitäten umfasst, wobei die Mikrokavitäten des ersten Stempels mit einer Polymerzusammensetzung beschichtet sind, die Polymilchsäure (PLA), Polymilchsäure-co-Glykolsäure-Copolymer (PLGA) oder eine Mischung davon umfasst;
b. Einfüllen des kristallinen Arzneistoffs in die beschichteten Mikrokavitäten;
c. Bereitstellen eines zweiten Stempels, wobei der zweite Stempel planar ist und auf mindestens einer Seite mit der Polymerzusammensetzung beschichtet ist, die Polymilchsäure (PLA), Polymilchsäure-co-Glykolsäure-Copolymer (PLGA) oder eine Mischung davon umfasst; und
d. Zusammenpressen der beschichteten Seiten des ersten und des zweiten Stempels, um den kristallinen Arzneistoff in den Kavitäten mit der Polymerzusammensetzung zu verkapseln, sodass sich eine Dünnschicht aus Mikrokammern bildet.

2. Verfahren nach Anspruch 1, wobei der erste und/oder der zweite Stempel mit einer Polymerzusammensetzungsschicht beschichtet sind, deren Dicke ungefähr 0,05 bis ungefähr 2 Mikrometer beträgt.

3. Verfahren nach Anspruch 1 oder Anspruch 2, wobei es ein Bereitstellen des ersten und des zweiten Stempels sowie ein Beschichten der Stempel mit einer Lösung, welche die Polymerzusammensetzung umfasst, und ein Verdampfen des Lösungsmittels der Polymerlösung umfasst, wobei die Polymerlösung möglicherweise eine Konzentration von ungefähr 0,1% bis ungefähr 25% oder von ungefähr 0,1% bis ungefähr 5% oder von ungefähr 0,5 bis ungefähr 2% oder ungefähr 1% aufweist.

4. Verfahren nach einem beliebigen der Ansprüche 1 bis 3, wobei es ein Entfernen der Dünnschicht aus Mikrokammern vom ersten Stempel umfasst.

5. Verfahren nach einem der Ansprüche 1 bis 3, wobei
(a) das Verfahren ein Zusammenpressen der beschichteten Seiten des ersten und des zweiten Stempels bei einem Druck von bis zu ungefähr 0,25 MPa (oder bis zu ungefähr 0,1 MPa) umfasst und das Verfahren keinerlei Schritt des Erhitzens der Polymerzusammensetzung umfasst,
wobei es möglicherweise weiterhin ein Trennen des ersten und des zweiten Stempels und ein Entfernen überschüssiger Polymerzusammensetzung vom ersten Stempel umfasst, wobei das überschüssige Polymer durch mechanische Einwirkung, beispielsweise durch Abkratzen, entfernt wird und wobei der Schritt des Entfernens überschüssiger Polymerzusammensetzung die Mikrokammern in separate Mikrokapseln in den Kavitäten des ersten Stempels unterteilt, und es weiterhin ein Entnehmen der Mikrokapseln aus dem ersten Stempel umfasst; oder
b) wobei der Schritt des Zusammenpressens der Stempel ein Erhitzen der Polymerzusammensetzung auf eine Temperatur umfasst, die höher als eine Temperatur ist, die 20 °C unter dem Schmelzpunkt der Polymerzusammensetzung liegt, und wobei das Verfahren ein Zusammenpressen der beschichteten Seiten des ersten und des zweiten Stempels mit einem Druck von mindestens ungefähr 0,1 MPa umfasst, wobei der Schritt des Zusammenpressens der Stempel während des Erhitzens die Mikrokammern in separate Mikrokapseln in den Kavitäten des ersten Stempels unterteilt, und er weiterhin ein Entnehmen der Mikrokapseln aus dem ersten Stempel umfasst;
wobei weiterhin möglicherweise
(i) der Schritt des Entnehmens der Mikrokapseln aus dem ersten Stempel Folgendes umfasst:
a. Bereitstellen eines planaren Substrats, das mit einem löslichen Klebstoff beschichtet ist;
b. Drücken des planaren Substrats auf den ersten Stempel, damit die Mikrokapseln an dem Flachträger anhaften;
c. Entnehmen der Mikrokapseln aus den Mikrokavitäten des ersten Stempels durch Trennen des planaren Substrats und des ersten Stempels; und
d. Auflösen des Klebstoffs, um die Mikrokapseln vom planaren Substrat zu trennen; und/oder
ii) das Verfahren weiterhin ein Formulieren der Mikrokapseln in eine injizierbare pharmazeutische Zusammensetzung mit einem oder mehreren pharmazeutisch unbedenklichen Hilfsstoffen umfasst.

6. Verfahren nach einem beliebigen der Ansprüche 1 bis 5, wobei die Mikrokapseln oder Mikrokammern einen Kern und eine Schale umfassen, wobei der Kern einen kristallinen Arzneistoff umfasst und die Schale Polymilchsäure (PLA) oder PLGA umfasst und wobei die Schale den Kern vollständig umschließt.

7. Verfahren nach Anspruch 6, wobei der Kern der Mikrokapseln oder Mikrokammern aus dem kristallinen Arzneistoff und gegebenenfalls Luft besteht.

8. Verfahren nach Anspruch 6 oder 7, wobei der kristalline Arzneistoff eine Löslichkeit von weniger als 1 mg/ml in Wasser bei 25 °C aufweist und einen Schmelzpunkt von mehr als ungefähr 200 °C aufweist.

9. Verfahren nach einem beliebigen der Ansprüche 6 bis 8, wobei es sich bei dem Arzneistoff um ein Kortikosteroid handelt, wobei das Kortikosteroid gegebenenfalls aus der Gruppe ausgewählt ist, die aus Dexamethason, Prednisolon, Betamethason, Prednison, Methylprednisolon, Budesonid, Hydrokortison, Triamcinolon und Fludrocortison besteht, oder wobei es sich bei dem Arzneistoff um ein Protein handelt, wobei es sich bei dem Protein gegebenenfalls um einen Wachstumsfaktor, ein Enzym, ein Cytokin, ein Chemokin oder ein biologischen Therapeutikum handelt;
wobei es sich bei dem Arzneistoff möglicherweise um Dexamethason handelt.

10. Verfahren nach einem beliebigen der Ansprüche 6 bis 9, wobei:
(a) die Schale einen Schmelzpunkt von ungefähr 50 °C bis ungefähr 200 °C aufweist; und/oder
(b) die Schale PLA-Homopolymer, PLGA oder eine PLA/PLGA-Mischung sowie möglicherweise Polycaprolacton (PCL) umfasst, wobei wahlweise:
i) die Schale aus PLA-Homopolymer besteht; oder
ii) die Schale aus PLA und PCL besteht und die Schale nach Gewicht mehr PLA als PCL aufweist.

11. Zusammensetzung zur langanhaltenden Freisetzung, die eine Mehrzahl an Mikrokapseln umfasst, welche mittels der Verfahren nach einem beliebigen der Ansprüche 5(a) bis 6 erhalten wurden oder erhalten werden können, oder die eine Dünnschicht aus Mikrokammern umfasst, welche mittels der Verfahren nach einem beliebigen der Ansprüche 1 bis 5(a) erhalten wurde oder erhalten werden kann.

12. Zusammensetzung zur langanhaltenden Freisetzung nach Anspruch 11, wobei
(a) die Zusammensetzung weiterhin einen oder mehrere pharmazeutisch unbedenkliche Hilfsstoffe oder Verdünnungsmittel umfasst, wobei die Zusammensetzung zur langanhaltenden Freisetzung möglicherweise PBS umfasst; und/oder
(b) wobei die Zusammensetzung injizierbar ist.

13. Zusammensetzung zur langanhaltenden Freisetzung nach Anspruch 11 oder Anspruch 12 zur Verwendung in der Behandlung einer Krankheit oder Störung,
wobei es sich bei der Krankheit oder Störung möglicherweise um eine entzündliche Krankheit oder Störung handelt, beispielsweise um Arthritis.

14. Arzneistoffreisetzende implantierbare oder injizierbare medizinische Vorrichtung, die eine Dünnschicht aus Mikrokammern gemäß der Begriffsbestimmung in Anspruch 11 umfasst oder daraus besteht.

## Revendications

1. Procédé de micro-encapsulation d'un médicament cristallin, comprenant :
a. la fourniture d'un premier tampon comprenant une pluralité de micropuits, les micropuits du premier tampon étant revêtus d'une composition de polymère comprenant un poly(acide lactique) (PLA), un copolymère poly(acide lactique-co-glycolique) (PLGA) ou un mélange de ceux-ci ;
b. le chargement du médicament cristallin dans les micropuits revêtus ;
c. la fourniture d'un deuxième tampon, dans lequel le deuxième timbre est plan et est revêtu sur au moins un côté par la composition de polymère comprenant un poly(acide lactique) (PLA), un copolymère poly(acide lactique-co-glycolique) (PLGA), ou un mélange de ceux-ci ; et
d. le pressage des côtés revêtus des premier et second tampons ensemble pour encapsuler le médicament cristallin dans les puits avec la composition de polymère pour former un film de microchambres.

2. Procédé selon la revendication 1, dans lequel les premier et/ou second tampons sont revêtus d'une couche de composition de polymère ayant une épaisseur d'environ 0,05 à environ 2 microns.

3. Procédé selon la revendication 1 ou la revendication 2, comprenant la fourniture des premier et second tampons, et le revêtement des tampons par une solution comprenant la composition de polymère et l'évaporation du solvant de la solution de polymère, éventuellement dans lequel la solution de polymère a une concentration d'environ 0,1 % à environ 25 %, ou d'environ 0,1 % à environ 5 %, ou d'environ 0,5 à environ 2 %, ou d'environ 1 %.

4. Procédé selon l'une quelconque des revendications 1 à 3, comprenant en outre le retrait du film de microchambres du premier tampon.

5. Procédé selon l'une quelconque des revendications 1 à 3, dans lequel
(a) le procédé comprend le pressage ensemble des côtés revêtus des premier et deuxième tampons à une pression allant jusqu'à environ 0,25 MPa (ou jusqu'à environ 0,1 MPa) et le procédé ne comprend pas une étape de chauffage de la composition de polymère,
éventuellement, comprenant en outre la séparation des premier et second tampons et l'élimination de l'excès de composition de polymère du premier tampon, dans lequel l'excès de polymère est éliminé par action mécanique, par exemple par raclage, et dans lequel l'étape d'élimination de l'excès de composition de polymère sépare les microchambres en microcapsules séparées dans les puits du premier tampon, et comprenant en outre l'élimination des microcapsules du premier tampon ; ou
(b) dans lequel l'étape de pressage des tampons ensemble comprend le chauffage de la composition de polymère jusqu'à une température qui est supérieure à une température qui est 20 °C en dessous du point de fusion de la composition de polymère, et dans lequel le procédé comprend le pressage ensemble des côtés revêtus des premier et second tampons à une pression d'au moins environ 0,1 MPa, dans lequel l'étape de pressage ensemble des tampons tout en chauffant sépare les microchambres en microcapsules séparées dans les puits du premier timbre, et comprenant en outre le retrait des microcapsules du premier tampon ;
en outre, éventuellement
(i) dans lequel l'étape de retrait des microcapsules du premier tampon comprend :
a. la fourniture d'un substrat plan revêtu d'un adhésif soluble ;
b. le pressage du substrat plan sur le premier tampon pour coller les microcapsules à la lame ;
c. le retrait des microcapsules des micropuits du premier tampon en séparant le substrat plan et le premier tampon ; et
d. la dissolution de l'adhésif pour séparer les microcapsules du substrat plan ;
et/ou
(ii) dans lequel le procédé comprend en outre la formulation des microcapsules en une composition pharmaceutique injectable avec un ou plusieurs excipients pharmaceutiquement acceptables.

6. Procédé selon l'une quelconque des revendications 1 à 5, dans lequel les microcapsules ou microchambres comprennent un noyau et une enveloppe, dans lequel le noyau comprend un médicament cristallin et l'enveloppe comprend un poly(acide lactique) (PLA) ou un PLGA, et dans lequel l'enveloppe encapsule complètement le noyau.

7. Procédé selon la revendication 6, dans lequel le noyau des microcapsules ou des microchambres est constitué du médicament cristallin et éventuellement d'air.

8. Procédé selon la revendication 6 ou 7, dans lequel le médicament cristallin a une solubilité inférieure à 1 mg/ml dans l'eau à 25 °C et a un point de fusion supérieur à environ 200 °C.

9. Procédé selon l'une quelconque des revendications 6 à 8, dans lequel le médicament est un corticostéroïde, éventuellement dans lequel le corticostéroïde est choisi dans le groupe constitué par la dexaméthasone, la prednisolone, la bétaméthasone, la prednisone, la méthylprednisolone, le budésonide, l'hydrocortisone, la triamcinolone et la fludrocortisone, ou dans lequel le médicament est une protéine, éventuellement dans lequel la protéine est un facteur de croissance, une enzyme, une cytokine, une chimiokine ou un agent thérapeutique biologique ;
éventuellement, dans lequel le médicament est la dexaméthasone.

10. Procédé selon l'une quelconque des revendications 6 à 9, dans lequel :
(a) l'enveloppe a un point de fusion allant d'environ 50 °C à environ 200 °C ; et/ou
(b) l'enveloppe comprend un homopolymère de PLA, un PLGA, ou un mélange PLA/PLGA, et éventuellement une polycaprolactone (PCL), éventuellement dans lequel :
(i) l'enveloppe est constituée d'homopolymère de PLA ; ou
(ii) l'enveloppe est constituée de PLA et PCL et contient plus de PLA que de PCL en poids.

11. Composition à libération prolongée comprenant une pluralité de microcapsules obtenues ou pouvant être obtenues par les procédés de l'une quelconque des revendications 5(a) à 6, ou comprenant un film de microchambres obtenu ou pouvant être obtenu par les procédés de l'une quelconque des revendications 1 à 5(a).

12. Composition à libération prolongée selon la revendication 11, dans laquelle
(a) la composition comprend en outre un ou plusieurs excipients ou diluants pharmaceutiquement acceptables, la composition à libération prolongée comprenant éventuellement du PBS ; et/ou
(b) dans laquelle la composition est injectable.

13. Composition à libération prolongée selon la revendication 11 ou la revendication 12, destinée à être utilisée dans le traitement d'une maladie ou d'un trouble,
éventuellement dans laquelle la maladie ou le trouble est une maladie ou un trouble inflammatoire, par exemple l'arthrite.

14. Dispositif médical implantable ou injectable à élution de médicament comprenant ou constitué d'un film de microchambres définies selon la revendication 11.
